(19) Europäisches Patentamt
European Patent Office
Office européen des brevets

(11) EP 4 811 364 A1

(12) EUROPEAN PATENT APPLICATION

(43) Date of publication:
**23.09.2026 Bulletin 2026/39**

(21) Application number: **25164204.7**

(22) Date of filing: **17.03.2025**

(51) International Patent Classification (IPC):
***G16B 40/20*** *(2019.01)* ***G16H 50/20*** *(2018.01)*

(52) Cooperative Patent Classification (CPC):
**G16H 50/20; G16B 40/20;** G16H 50/70

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **FUJITSU LIMITED**
**Kawasaki-shi, Kanagawa 211-8588 (JP)**

(72) Inventors:
- **BUI, Van Tu**
  **Slough, SL1 2BE (GB)**
- **SULIMAN, Mohamed Abdalla**
  **Slough, SL1 2BE (GB)**
- **HALDAR, Aparajita**
  **Slough, SL1 2BE (GB)**
- **GEORGESCU, Serban**
  **London, W13 8NH (GB)**

(74) Representative: **Haseltine Lake Kempner LLP**
**Cheapside House**
**138 Cheapside**
**London EC2V 6BJ (GB)**

Remarks:
Amended claims in accordance with Rule 137(2) EPC.

# (54) A METHOD FOR CLASSIFYING BIOLOGICAL DATA

(57) A computer implemented method for training a graph neural network, GNN, to classify a medical condition of samples using a biological dataset, comprising loading the biological dataset, the biological dataset comprising samples and the samples comprising features and a classification of the medical condition, extracting relationships between the features from a knowledge base, constructing, for the samples, graphs comprising nodes and edges between the nodes, wherein the nodes correspond to the features and the edges correspond to the relationships; and training the GNN with the graphs to provide the classification of the medical condition for the samples.

200
210
Dataset
210
Knowledge base
Graphs
220
GNN training
230
Prediction
240

Figure 2

EP 4 811 364 A1

## Description

### Technical field

The present invention relates to methods for training a graph neural network. In particular, the present invention relates to methods for training a graph neural network to classify a medical condition.

### Background

The rise of computer-assisted systems in medical diagnosis and prognosis marks a transformative period in healthcare, driven by rapid advancements in artificial intelligence (AI) and Deep Learning (DL). These technologies have ushered in a new era of precision and efficiency, enabling healthcare professionals to leverage vast amounts of patient data to deliver highly accurate and personalized diagnoses in a timely manner. However, most recent medical AI/DL applications are centered around only the image domain, according to the World Economic Forum 2025 findings. These unbalanced developments are also observed in other sectors, not just in health care.

Despite the transformative impact of deep learning on text, audio, and image datasets, its dominance in tabular data, especially in the medical domain where data are often scarce, remains less clear. Statistical and tree-based methods still remain dominant for tabular datasets. The main reason for the lack of a wide adaptation of DL on tabular data may stem from the inherent challenges of tabular data itself, for instance, challenges include: (i) tabular dataset often contains mixed type data e.g. continuous, categorical, date time; (ii) is often moderate in size; (iii) the meaning of cell values may dependent on contextual metadata e.g. column names; and (iv) may be invariant/equivariant with respect to the column order, encouraging the modeling methods to also have such invariance.

Biological tabular data, such as copy number variants (CNVs) and ribonucleic acid (RNA) expression profiles, and clinical information, are used for medical research and healthcare innovation. Unlike multi-dimensional data such as images, these datasets are often limited and complex but can offer vital insights into biological processes and diseases.

Early machine learning methods such as multi-perceptron layer (MLPs) based neural networks (NNs) face challenges with biological tabular data. MLPs treat features independently, making it hard to capture complex inter-dependencies, especially with mixed numerical and categorical variables. They also require extensive preprocessing, which may introduce errors and increase computational demands. Rule-based models, though popular, are sensitive to hyperparameter tuning and struggle with extrapolation and capturing complex non-linear relationships. Both MLPs and rule-based models are further limited by the scarcity of labelled data in medical applications, restricting their learning potential.

It is therefore desirable to improve data manipulation and modelling for classifying and/or predicting/determining medical conditions. In particular, it may be desirable to improve machine learning methods specifically tailored for biological tabular data.

### Summary of the invention

The present invention is defined in the independent claims, to which reference should now be made. Advantageous features are set out in the sub claims.

In an aspect there is provided a computer implemented method for training a graph neural network, GNN, to classify a medical condition of samples using a biological dataset, comprising loading the biological dataset, the biological dataset comprising samples and the samples comprising features and a classification of the medical condition, extracting relationships between the features from a knowledge base, constructing, for the samples, graphs comprising nodes and edges between the nodes, wherein the nodes correspond to the features and the edges correspond to the relationships; and training the GNN with the graphs to provide the classification of the medical condition for the samples.

In another aspect there is provided a computer implemented method for providing a classification of a medical condition from biological data, comprising inputting a new sample, the new sample comprising features, loading a knowledge base, the knowledge base comprising relationships between the features in the sample, constructing, for the sample, a graph comprising nodes and edges, wherein the features form the nodes and the relationships form the edges, inputting the graph into a graph neural network, GNN, to classify the condition, wherein the GNN is trained according to any preceding claim, and outputting the classification of the medical condition for the new sample.

In yet another aspect there is provided a computer program which, when run on a computer, causes the computer to carry out a method for training a graph neural network, GNN, to classify a medical condition of samples using a biological dataset, comprising loading the biological dataset, the biological dataset comprising samples and the samples comprising features and a classification of the medical condition, extracting relationships between the features from a knowledge base, constructing, for the samples, graphs comprising nodes and edges between the nodes, wherein the nodes correspond to the features and the edges correspond to the relationships, and training the GNN with the graphs to

provide the classification of the medical condition for the samples.

In yet another aspect there is provided an information processing apparatus for training a graph neural network, GNN, to classify a medical condition of samples using a biological dataset comprising a memory and a processor connected to the memory, wherein the processor is configured to load the biological dataset, the biological dataset comprising samples and the samples comprising features and a classification of the medical condition, extract relationships between the features from a knowledge base, construct, for the samples, graphs comprising nodes and edges between the nodes, wherein the nodes correspond to the features and the edges correspond to the relationships, and train the GNN with the graphs to provide the classification of the medical condition for the samples.

## Brief description of the Figures

Reference is made, by way of example only, to the accompanying drawings in which:

**Figure 1** shows steps of a method for training a graph neural network, GNN, to classify a medical condition of samples using a biological dataset;
**Figure 2** shows a block diagram of a method for classifying a medical condition from a dataset;
**Figure 3** shows an example of a graph and a corresponding knowledge base for the graph;
**Figure 4** shows an example node construction which may be used with the methods described herein;
**Figure 5** shows an example structure of a GNN which may be used to implement the methods described herein;
**Figure 6** shows another block diagram for classifying a medical condition from a dataset;
**Figure 7** shows an example extract of a copy number variant dataset which may be processed using the methods described herein;
**Figure 8** shows an example extract of an RNA-sequence dataset which may be processed using the methods described herein;
**Figure 9a** provides an example extract of a clinical dataset 900a which may be processed using the methods described herein.
**Figure 9b** shows an illustrative example of tabular dataset to graph conversion process for a subset of clinical data;
**Figure 10** shows Precision-Recall (PR) curves and Average Precision (AP) of the methods described herein along with three benchmarks;
**Figure 11** shows the k most importance features identified from the methods described herein;
**Figure 12** shows contributions of each class and knowledge base for the methods described herein;
**Figure 13** shows example graphs constructed from knowledge bases;
**Figure 14** shows a block diagram of an information processing apparatus or a computing device or server which may implement the methods described herein.

## Detailed description

Within the health care sector, the existence of biological tabular data (BioTD) is often abundant, from electrical health records to genetic data such as copy number variation (CNV) and RNA expressions. Such data, and further clinical data including patient demographics, laboratory results, and medical history, may provide context which enriches the interpretation of imaging and molecular data, thereby improving the prediction capabilities of AI models. Molecular data (e.g., CNVs, RNA, proteins) may reveal core health and disease mechanisms, aiding in cancer subtype identification, biomarker discovery, and personalized medicine. Clinical data, detailing patient history and treatment outcomes, may be used for diagnosis, treatment customization, and understanding patient health, supporting tasks like cancer subtyping and risk prediction.

CNV data may offer valuable insights into genetic alterations often associated with medical conditions/diseases, and its inclusion in AI models may facilitate a more comprehensive understanding of genetic contributions to health conditions. RNA sequencing data may further augment the predictive power of AI model by revealing the transcriptomic landscape, which may reflect the functional state of genes and may uncover disease mechanisms that may not be apparent through imaging alone.

Collectively, diverse data modalities may empower AI models not only to predict outcomes with greater precision but also to uncover novel insights into disease mechanisms. The inventors found that these modalities may pose even greater challenges in modeling tabular data with deep learning. Medical data may be scarce due to the sensitive nature and strict requirements for patient privacy preservation. Furthermore, the high time and labor cost of experiments may also limit the amount of annotated data. On the other hand, the inventors found that CNV and RNA data tends to have a vast number of genes, which may significantly reduce the samples-to-features ratio, and therefore, may worsen the risk of overfitting when training a DL model.

Early ML approaches for modelling tabular data are based on feature selection, regression or rule-based shallow

methods such as decision trees and random forest. Known methods for modelling biological tabular data may therefore be categorized into 2 types of models: rule-based or deep learning.

Rule-based methods (e.g. decision tree, random forest) aim to split data based on feature values, creating a tree where each internal node represents a decision rule. Deep learning methods may use multi-layer neural networks to automatically learn and extract features for target prediction. Current deep learning methods for biological data predominantly uses Multi-Layer Perceptron (MLP) models where all neurons within a layer are fully connected and weighted edges between neurons are learnt during training through backpropagation.

Unlike image or text domains where data is often abundant, biological tabular data is often scarce. Certain biological datasets even have the number of samples an order smaller than the number of features. This may cause problems for MLP-based methods, such as overfitting, shortcut learning, and spurious correlation. Meanwhile, tree-based methods often do not generalise well and are sensitive to noisy and complex data inter-relationships. The model XGBoost [Tianqi Chen and Carlos Guestrin. Xgboost: A scalable tree boosting system. In Proc. ACM SIGKDD, pages 785-794, 2016*]* may further improve accuracy and robustness by sequentially building trees that correct the errors of the previous ones. Being also a computationally efficient and interpretable algorithm, XGBoost may be a preferred choice of known methods for modelling small to medium-tabular data.

Deep learning methods may handle complex, high-dimensional relationships and scale to large datasets but are less interpretable and often require substantial training data. In the healthcare domain, MLP-based methods are widely used on tabular data, often bootstrapped with an image model (whose training data is more abundant) in a multimodal fashion. Transformer-based models, meanwhile, leverage attention mechanisms to capture complex dependencies between features at the cost of computational complexity.

Challenges such as overfitting and spurious correlations are persistent issues that are exacerbated by insufficient training. The use of self-supervised learning (SSL) could alleviate these challenges, but may require a large amount of extra data, often unlabelled and in different distributions, which in turn, add complexity to the learning task. Current MLP-based methods are known for not performing well on biological data, especially when sample number is small, see for instance Shwartz-Ziv, R. and Armon, A., 2022. Tabular data: Deep learning is not all you need. Information Fusion, 81, pp.84-90. It was shown in Shwartz-Ziv, R 2022, that the MLP method even underperforms rule-based methods.

Furthermore, MLP methods may cause overfitting and spurious correlation. Since all neurons in MLP-based methods are connected, the number of parameters to be optimized is large. With limited training data, the model has fewer examples to learn the underlining patterns that help generalise on unseen data. Instead, the model may mistakenly identify noise or insignificant patterns that explain the training data well but not on unseen data. A sign of this problem may be observed during training with a large gap between the training and validation performance metrics. While employing regularization (e.g. L1/L2 regularization to penalize large weights, dropout) or data augmentation could alleviate over-fitting/spurious correlation, these techniques come with their own drawbacks - regularization could cause underfitting and it is often difficult to define a meaningful data augmentation strategy for tabular data.

Graph deep learning models, such as Graph Neural Networks (GNNs), are becoming increasingly popular models. The inventors found that strengths of GNN models include the ability to capture complex relationships and interdependencies between features in tabular data. Unlike MLP methods in which all neurons are connected, GNN models may perform message passing within nodes connected by an edge only. Overfitting may, therefore, be constrained through an inductive bias defined by the graph edges.

As described herein, graph-based models may provide an alternative for analysing biological tabular data. For instance, graph-based models may represent features and/or entities as nodes and their relationships as edges, capturing dependencies often missed in traditional formats. In general, Graph Neural Networks (GNNs) aggregate information from connected nodes, learning complex interactions and enhancing performance. GNNs may also reduce redundancy, emphasize meaningful interactions, and help prevent overfitting (compared with other models such as MLPs), which may be useful for high-dimensional datasets. GNNs may also improve model explainability by tracing feature contributions to predictions. The quality of the graph may depend significantly on the construction method, which may for example, be either a rule-based method, a learning-based method, or a knowledge-based method.

Rule-based methods may use predefined criteria to define nodes and edges, often representing columns as nodes and establishing edges through similarity metrics. For instance, some methods use k-Nearest Neighbors (kNN) to link each node to its k closest neighbors, which is effective for clustered data but can be computationally intensive and sensitive to k's choice. Alternatively, a similarity threshold may be applied, which is also sensitive to the choice of the threshold. Other methods create dense graphs by connecting all nodes, resulting in highly computational approaches, or link nodes only if they share identical values for a specific feature, which may lead to isolated clusters in the graph, especially if many unique feature values exist.

Learning-based methods may focus on discovering the optimal graph structure that reflects data relationships. Techniques include using kernel functions for node feature, neural networks to predict edges based on edge features, and directly learning the adjacency matrix as parameters. These methods may face challenges such as difficulty in parameter selection, overfitting due to noise, and the high cost of efficiently learning matrix parameters, respectively.

The methods described herein may convert biological tabular data into graphs using external knowledge sources. The methods described herein may directly transform tabular data into graphs. Columns in the tabular data may be converted to nodes and may be connected based on relationships identified by external sources, such as shared contributions to a condition/disease. This may result in a collection of graphs suitable for GNN modelling and prediction, setting the methods apart from MLP-based approaches.

The following non-limiting definitions may be useful for understanding terminology used throughout the description.

**Biological data** - Biological data includes omics and phenotypic data of living organisms.

**Sample/Observation** - these terms may be used interchangeably and may refer to a row of tabular data. For example, in the TCGA-BRCA dataset discussed herein, each sample may include a record of one patient.

**Features** - may refer to column headings of the tabular data. Each sample in the table may include a set of features for each column heading. Each column heading may comprise a feature label and each column may comprise feature values.

**MLP (Multi-Layer Perceptron)** - a type of artificial neural network consisting of multiple layers where each layer is fully connected to the next.

**GNN (Graph Neural Network)** - a type of artificial neural network which may be designed to process and learn from graph-structured data by aggregating and transforming information from a node's neighbours iteratively.

**Nodes and edges** - may describe two components of a graph $G = \{V, E\}$ where $V = \{v_1, v_2, ..., v_n\} \in R^{n \times d}$ may include the graph's n nodes (or vertices) each for example having d dimensional features, E may include a set of edges representing connections between pairs of nodes. Each edge $(u, v) \in E$ may connect two nodes $u,v \in V$.

**Inductive bias** - may include a set of assumptions a model makes to generalize from the training data to unseen data. These assumptions may guide a learning algorithm in choosing one hypothesis over others when multiple hypotheses may explain training data equally well, thereby influencing the training data's ability to perform well on new data. The concept of inductive bias may extend to the predefined set of rules that a model should follow during the learning process.

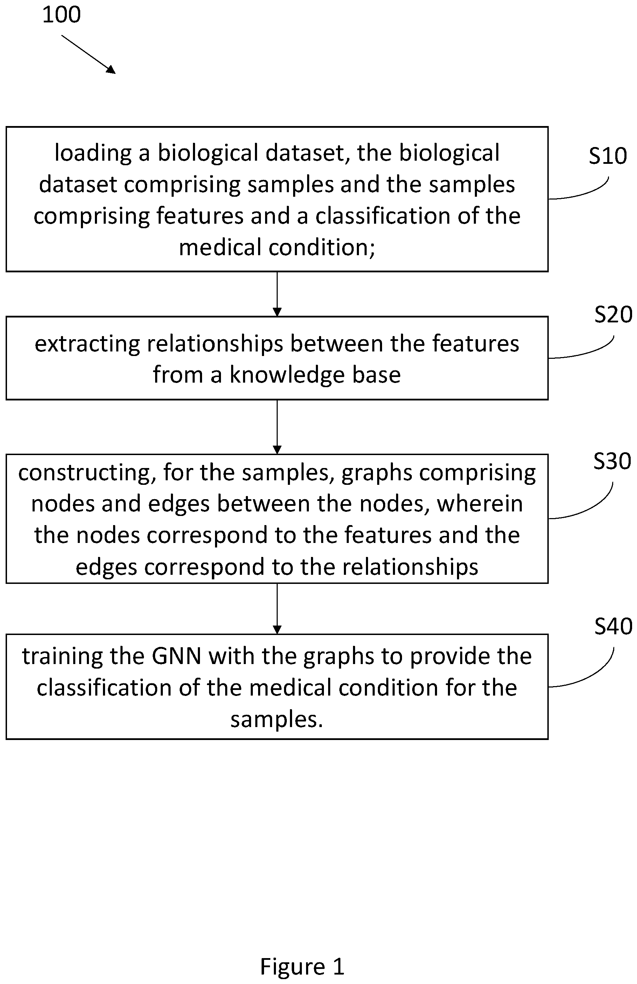

**Figure** 1 shows steps of a method 100 for training a graph neural network, GNN, to classify a medical condition of samples using a biological dataset. The method may be a computer implemented method.

In a loading step S10, a biological dataset may be loaded. The biological dataset may comprise samples. The samples may comprise features and a classification of the medical condition associated with the sample (such as a cancer subtype). In particular, the medical condition may be breast cancer and the classification may determine a subtype of breast cancer such as at least one of Luminal A, Luminal B, Her2-enriched and Basal-like breast cancer. A sample may be data taken from/related to a patient and/or person/human and/or animal.

The features may include feature labels. The dataset may further include attributes of the features. In an example, the feature labels may be gene labels, and the attributes of the features may be copy number variants, CNV, values of the gene labels and/or RNA sequence numbers. Additionally or alternatively, the feature labels may be are clinical labels and the attributes may be health record entries. That is, the attributes for the clinical data may comprise medical data/ answers for each feature label.

The feature labels may be encoded using an embedding layer. The feature labels may comprise words and the embedding layer may convert the words into a feature label vector within an embedding space. The attributes may be encoded using a linear layer. The attributes may comprise numerical values and the linear layer may convert the numerical values into an attribute vector within another embedding space. The dimension of the embedding space generated by the embedding layer and the other dimension of the other embedding space generated by the linear layer may be the same size.

In an extracting step S20, relationships between the features may be extracted from a knowledge base. The knowledge base may be an external data source. The knowledge base may share features (and/or feature labels) with the biological dataset. The knowledge base may be loaded before extracting the relationships.

The relationships between features may indicate that the features are associated according to the knowledge base. For example, the association between features may comprise at least one of: the features sharing the same classification of the condition, the features sharing a same biological pathway group, and/or the features sharing a molecular interaction.

The knowledge base may comprise more than one association between features. However, during the extracting step, only one type of association may be extracted, for example, only the relationship indicating that features share the same classification, etc., may be extracted. In another example, the knowledge base may comprise only one type of association (type of relationship).

In a constructing step S30, for the samples, graphs comprising nodes and edges between the nodes may be constructed. The nodes may correspond to the features and the edges may correspond to the relationships. A (single) graph may be constructed for each sample.

In an example where the features include feature labels and the dataset further includes attributes of the features,

the nodes may comprise the feature labels and the attributes of the features. The features and attributes may be encoded using the embedding layer and linear layer respectively, and the node may be formed by combining the feature label vector and the attribute vector. For example, the feature label vector and the attribute vector may be concatenated.

Features present in both the biological dataset and the knowledge base may be used to construct the graphs. Preferably any features not contained within the knowledge base may be excluded from the graphs. For example, the knowledge base may act to filter the biological dataset so that only features in the knowledge base are included in the construction of the graphs for the samples.

Nodes and/or edges from one or more of the graphs may be removed before the graphs are used to train the GNN. For example, a random node or nodes and/or random edge or edges may be removed from one or more of the graphs. The node(s) and/or edges removed from each graph may be different. Removing nodes and/or edges from the graphs may increase diversity of the biological dataset for training the GNN.

In a training step S40, the GNN may be trained with the graphs to provide the classification of the condition for the samples.

The GNN may be trained by inputting the graphs into the GNN and adjusting weights of the GNN to predict correct classifications for the graphs. The GNN may be trained using supervised learning. For instance, each graph may be associated with the correct classification of the condition and the GNN may be trained in a supervised learning manner by updating weights based on a comparison of the predicted classification of condition and the correct classification of the condition for that graph. The GNN may be trained used backpropagation.

In an optional outputting step, the method may further comprise outputting the classification of the condition for at least one of the samples.

The graphs may form a graph set (corresponding to the knowledge based used). The method may further comprise, constructing another graph set using the features from the biological dataset and relationships from another knowledge base; training another GNN with the other graph set; and combining the classification of the condition of the GNN and the other GNN into a final (which may otherwise be referred to as a global) classification of the condition.

For instance, the (first) knowledge base may comprise relationships associating features sharing the same classification of the condition and the other (second) knowledge base may comprise relationships associating features sharing a same biological pathway group, and/or features sharing a molecular interaction.

The classification of the condition may be combined using a classifier. The classifier may include a linear layer. The classifier may be trained in a corresponding manner to the GNN(s). For instance, the classifier may be trained using backpropagation.

In the example with relationships being extracted from the knowledge base and the other knowledge base features which are present in both knowledge bases may be used for constructing the graphs. For example, any features not contained within the knowledge base and the other knowledge base may be excluded from the graphs.

In another example, further relationships between the features may be extracted from another knowledge base. The edges of the graphs may include the further relationships. This method may provide an alternative to training multiple GNNs. While relationships from multiple knowledge bases may be extracted, the relationships for features may be combined in the same edge between features.

The knowledge base may comprise further information on the relationships between features. The edges may include the further information as edge attributes. The further information may include a type of disease/the medical condition which links the two features and/or may indicate the strength of the relationship between the features.

The biological dataset may be a tabular dataset and may therefore be referred to as biological tabular data. The sample names may form a samples column in the dataset. The features (or feature labels) may form feature columns, and the feature columns may comprise the attribute of the features so that the attributes of the features form a row for each sample.

The biological tabular data may refer to omics and phenotypic data in tabular format. As described above, each row may comprise feature attributes for a sample (or observation) and each column may be comprise a feature (biological attribute). The tabular data may be either homogeneous (all features may be of the same type) or heterogenous (features may be of varied types). Attributes may be convertible to numerical values, e.g. if a feature/column has name "gender" and attribute (value) of either "Male" or "Female", the attribute may be converted to numerical value 0 or 1 respectively.

The method may further comprise an optimization step. The optimization step may comprise training multiple types of GNN with different preset parameters and selecting the GNN and parameters with a highest performance metric as the GNN used classification of the condition. The GNN and parameter selection may be determined using, for example a grid search technique. The multiple types of GNN may be, for example, Graph Convolutional Network (GCN) models, Graph Attention Network (GAT) models, and Sample and Aggregate Convolution (SAGEConv) models. Types of present parameter may include model architecture parameters such as: number of Layers of the GNN, hidden dimension (the size of the node embedding), aggregation function, activation function, dropout rate, batch normalization and residual connections, and/or training hyperparameters such as: learning rate, optimizer type (e.g., Adam), loss function, weight decay and gradient clipping. Other parameters may also be considered such as graph sampling parameters (e.g., batch

size) and training strategy parameters (e.g., number of epochs).

In an aspect, a computer implemented method for providing a classification of a medical condition (for example for cancer subtyping prediction) from biological data may also be provided, the method comprising: inputting a new sample, the new sample comprising features; loading a knowledge base, the knowledge base comprising relationships between the features in the sample; constructing, for the sample, a graph comprising nodes and edges, wherein the features form the nodes and the relationships form the edges; inputting the graph into a graph neural network, GNN, to classify the condition; and outputting the classification of the condition for the new sample. The GNN may be trained according to any of the above examples. The knowledge base may be the same knowledge base as used to train the GNN. Known methods to model tabular data with GNNs may be to convert the whole table into a large graph whose nodes correspond to either rows (samples), columns (features) or both (heterogeneous), while edges represent node similarity given a predefined distance metric. Inductive bias may be obtained intrinsically from the data itself, which is susceptible to noises and outliers.

In contrast, the methods described herein may leverage knowledge, from for example external sources (that is sources external to the biological dataset) to define the graph edges, offering a more accurate inductive bias for model learning. The abundant external knowledge (henceforth knowledge base, or KB) in the biological domain may also provide an added advantage.

As described in the background above, MLP based models may not perform well with biological data, especially when the sample numbers are small. Furthermore, with small samples, MLP models may overfit the training data and generate spurious correlations.

In contrast, by using knowledge bases (for instance external knowledge sources/ external domain knowledge which may be abundant in literature) to define node connections (e.g., edges) the methods described herein may strengthen the inductive bias during model learning and reduce overfitting. By introducing an inductive capability into modelling methods, a potential data-limitation problem in biological tabular data may be addressed and identification/-classification of conditions may be improved.

Furthermore, the inventors found that column headers (e.g., feature labels) in tabular data are not utilized in current methods. MLP methods treat each column equally, ignoring the column headers available in tabular data. In contrast, the methods described herein may enable the feature labels (e.g., column ID) to be optionally encoded in the graph node features.

In an example application of the methods described herein, where the number of nodes in the constructed graph is very large, certain nodes might be dropped (either randomly or selectively), resulting in an efficient data augmentation for more robust model training. The node dropping strategy may be memory efficient, enabling training on smaller GPUs. In such cases, the column ID/header (e.g. gene name) may be encoded as part of the node feature, so that the graph is permutation invariant.

The methods described herein introduce a process for modelling tabular data by, for example, converting each (1-D) sample into a graph. In particular, a (row) sample of biological tabular data may be converted into a view invariant graph and these graphs may be used to train a GNN with standard message parsing architecture.

The methods may allow for the implementation of the GNN (which generally cannot be implemented for raw tabular data), which may lead to superior performance compared to MLP-based methods. The methods may be particularly effective for tabular data with a small number of rows compared with the number of columns.

Thus, the methods described herein may:

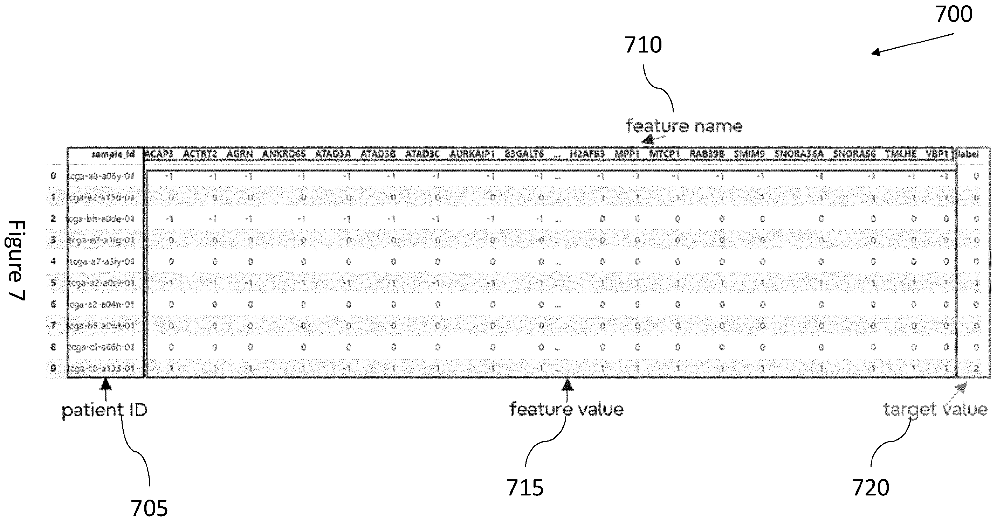

- Convert biological tabular data samples to graphs and model them with graph neural networks, in contrast with existing DL methods mostly based on multilayer perceptrons (MLP) or attention mechanism.
- Achieve state-of-art performance on three real-life BioTD datasets, covering three distinct modalities (CNV, RNA, Clinical) when compared with various DL and statistical methods (see Figures 7 to 13).

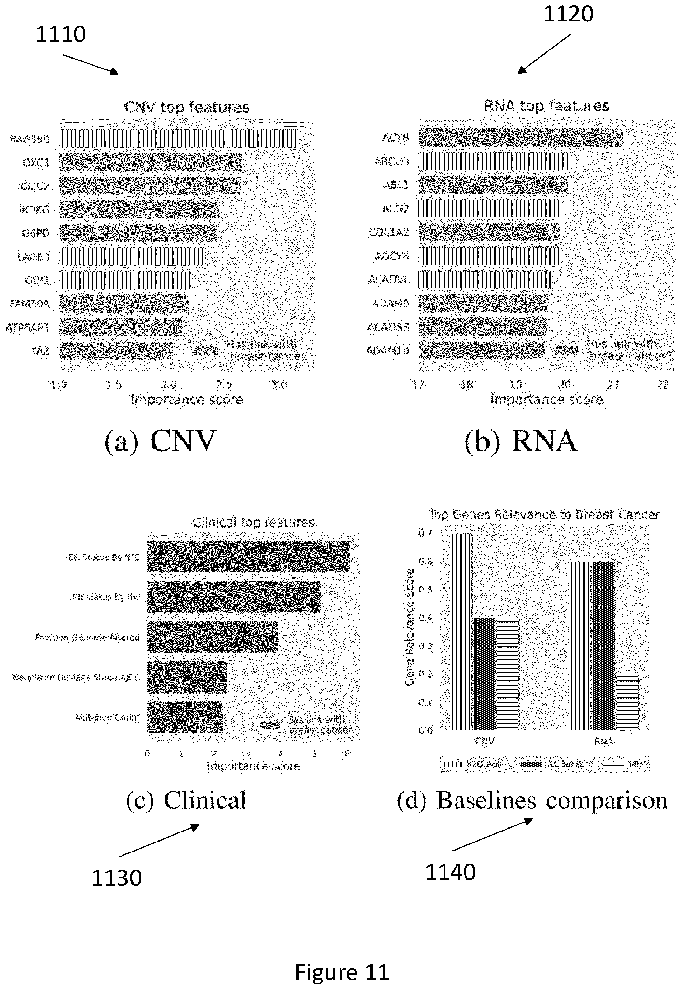

Furthermore, a graph-based interpretability example is provided herein which may highlight key contributing components and important features that may underpin predictions by the GNN (see Figure 11)

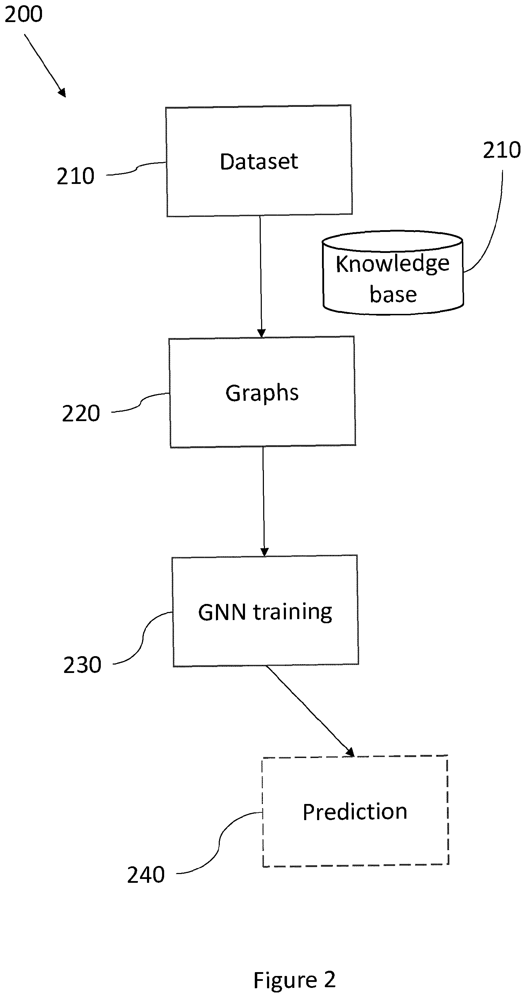

**Figure 2** shows a block diagram 200 of an architecture for classifying a medical condition of samples using a dataset. For example, the architecture may perform the methods described in Figure 1. The dataset may be, for example a biological dataset. The dataset may be presented in a tabular form with samples, or observations, as rows and features of each sample as columns.

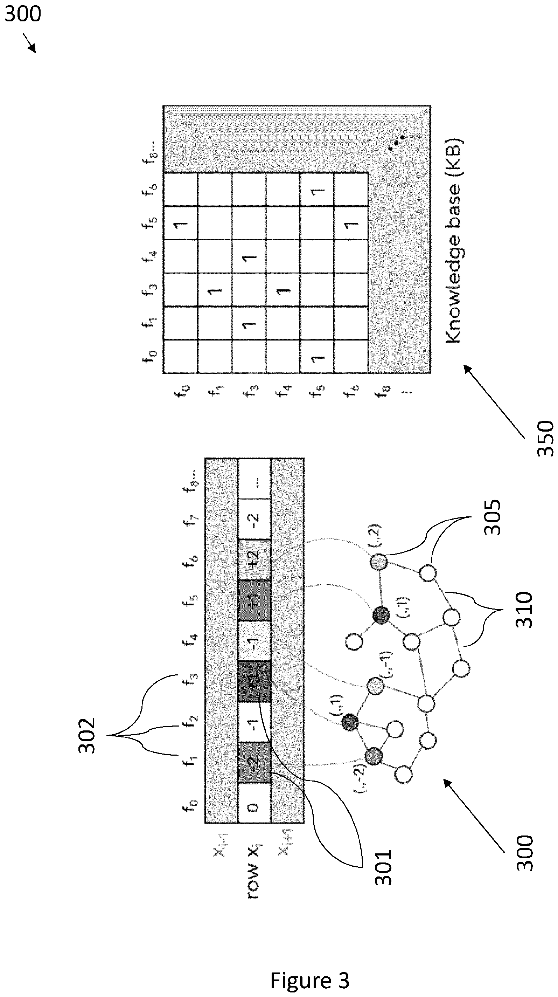

The method described herein may extract relationships between the features from a knowledge base (KB) 210. A KB may be a square matrix where the value at (i, j) represent the connection between feature i and feature j. An example format of a knowledge base is provided in Figure 3.

The inventors found that, in practice, the number of features in the KB may not be the same as the number of features in the dataset. In this case, the table features that also appear in the KB may be transformed into graphs while other features (columns) may be discarded. The relationship between features, which may be referred to as feature

connections or associations, may come from established facts, or reasonable assumption. If a connection between two features is 1-way, then the graph may be referred to as a directed graph. Otherwise, if the KB connection matrix is symmetrical across the diagonal, all constructed graphs may be undirected.

The KB may not be exhaustive. That is, the knowledge base may not share all the same features as the samples. However, as shown in the example Figures 7 to 9, the KB may only cover a fraction of the features available in the tabular data but still provide effective results. The more accurate/complete the KB, better performance tends to be achieved.

There may exist multiple KBs for a dataset, since two features may have more than one type of connection. Taking genes as example features, a relationship may form between genes if, for example, two genes relate to the same disease, the same pathway or have the same molecular interaction. An example is gene BRCA1 and CDH1 in CNV/RNA data - the genes may be considered connected under the disease perspective in the MoNDO KB [Mungall et al. The monarch initiative: an integrative data and analytic platform connecting phenotypes to genotypes across species. Nucleic acids research, 45(D1):D712-D722, 2017.] (they are known to link to the same disease), but not connected under the pathway perspective in the Reactome KB [Fabregat et al. The reactome pathway knowledgebase. Nucleic acids research, 46(D1):D649-D655, 2018.*]*.

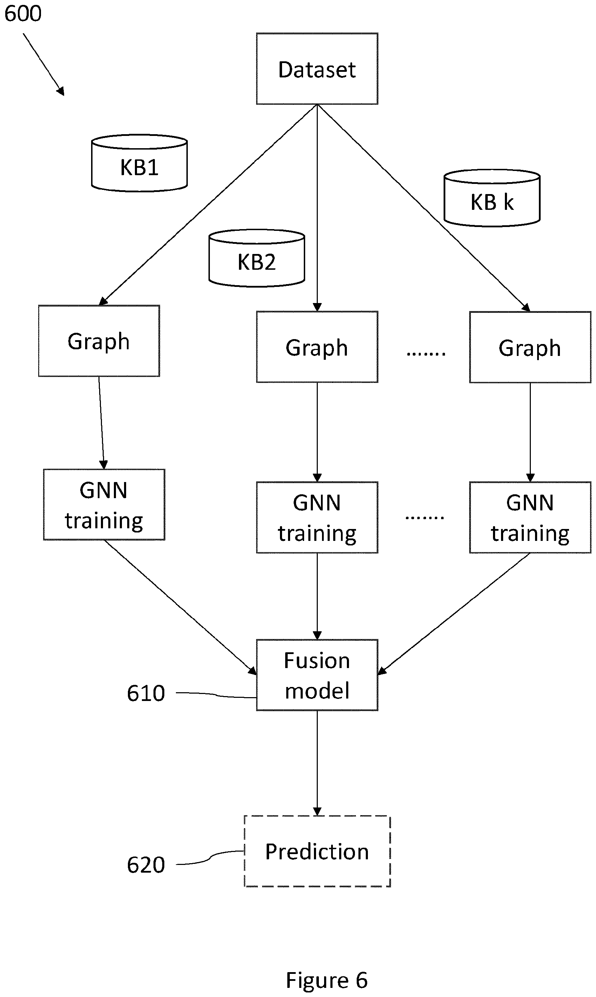

The example in Figure 2 shows a single knowledge base. The relationship between features may be extracted from a single knowledge base. However, if for example multiple knowledge bases are available, multiple graph sets may be constructed one for each KB (see for instance Figure 6), or a graph set with a multi-attribute KB may be constructed where value at (i,j) is a multi-dimensional feature and multiple relationships between features may be included in an edge between the features.

Graphs 220 may be constructed for the samples in the dataset. That is, the data table may be transformed into a set of graphs. A graph may be constructed for each sample (observation) in the dataset and the graph neural network modelling technique may be applied for classification of the medical condition.

Features in a sample may represent a node in a graph, while the edges connecting nodes may be represented by a relationship between the nodes extracted from the knowledge base. The knowledge base may be an external source of information. That is, the knowledge base may be a source of data/information external to the dataset. Figure 3 shows an illustrative example of a graph and a knowledge base.

The graphs may be input into a graph neural network (GNN) for training the GNN (i.e. GNN training 230). The GNN may be trained to provide a classification of the condition for the samples. That is, each graph may correspond to a sample and each sample may comprise a classification of the condition.

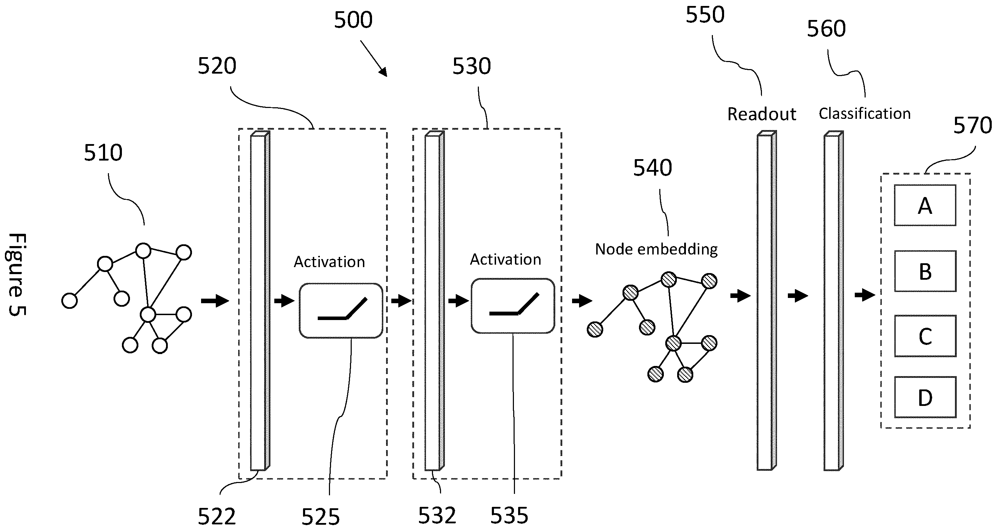

The GNN may be trained so that, if it receives a new sample as an input, it may provide a classification of the condition that the new sample presents (e.g., prediction 240). Detailed examples for the training and prediction process are provided in Figure 7 to 13 for classifying/predicting breast cancer types from genes. More detail of the structure of a GNN which may be used with the architecture in Figure 2 is provided in relation to Figure 5.

The methods described herein may convert biological tabular data to graph-structured data by leveraging external knowledge source(s) that define the connections between features (e.g., columns in the tabular data). For example, in a Copy Number Variations (CNV) dataset, each feature (column name) may refer to a gene. A relationship between features, extracted from the external knowledge source (i.e., knowledge base) may associate the features with the classification of a condition. For example, a relationship may exist between two genes if they are both responsible for the classification of the same condition, e.g., development of the same disease, or are related to the same pathway or molecular structure. If a relationship exists between two features the features may be defined as associated/linked/connected. Thus, the methods described herein may transform tabular data into a collection of graphs on which Graph Neural Network (GNN) learning models may be trained/applied.

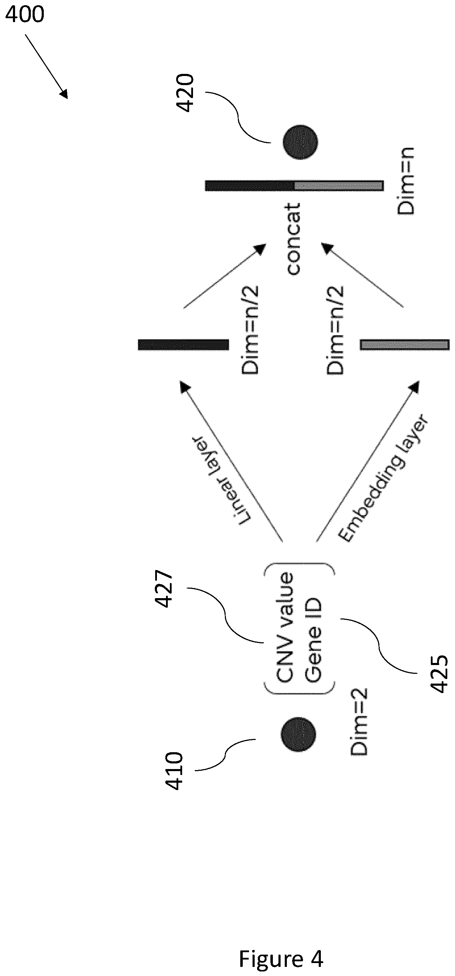

**Figure 3** shows an example of a graph 300 and a corresponding knowledge base 350 for the graph. The graph is an example of a graph which may be constructed using the methods described herein. The knowledge base is an example of a KB in which relationships between features may be extracted. The Figure shows an example graph for a sample $x_i$ in a tabular dataset. The cell values (i.e. attributes 301 in feature columns 302) may form nodes 305. The edge connections may be extracted from the corresponding knowledge base. An edge 310 may be drawn between nodes if the knowledge base indicates a relationship between the features forming the nodes. The (.) notation in the graph refers to other information which may be incorporated alongside the cell value in a node, for example the feature label (as an embedding) or a feature index/ID. Figure 4 provides an example of a node construction.

Not all cells (features/ attributes) at row $x_i$ may appear in the graph as in the case of f2 and f7 in this example as they are not available in the KB above. Additionally or alternatively, features may not be included, in this example, f0, according to certain assumptions e.g. the value of f0 is not meaningful for the subsequent modelling task. For example, if a feature is denoted with an attribute value of zero, which indicates that feature is not related to the medical condition associated with the sample, that feature may not be included in the graph. An example may be in copy number variant data with a zero value indicating no deviation, and so may not be included in the graph.

Initially, all graphs constructed according to the same knowledge base may have the same topology because their edges come from the same KB. However, certain nodes may be dropped to diversify the training data. Data

augmentation may additionally or alternatively be applied during model training that changes the graph edge structure. For instance, some edges between graphs may be dropped. This may diversify the training set and improve modelling.

The following algorithms, 1 2 and 3 provide exemplary step by step methods for constructing graphs with a knowledge base. In the below algorithm the graph is referred to as an 'X2Graph'. The steps in the methods below may be integrated into the methods described in relation to Figure 1. Although described together, each step below may be taken in isolation and included in the method described in relation to Figure 1.

**Algorithm 1:** X2Graph algorithm.

**Input:** Tabular dataset $\mathcal{D} = \{\mathbf{X}_D, \mathbf{S}_D\}$ where $\mathbf{X}_D \in \mathbb{R}^{N \times M_0}$ is the value matrix of N rows and $M_0$ columns; $\mathbf{S}_D$ is the $M_0$-length list of column names

**Output:** Graph set $\{\mathbf{G}_i\}, i \in [1, N]$

**Data:** Knowledge graph $\mathcal{G} = \{\mathbf{X}_G, \mathbf{S}_G\}$ where $\mathbf{X}_G \in \mathbb{R}^{M_G \times M_G \times \cdots}$ is the square connection matrix on the first two dimensions and $\mathbf{S}_G$ is the $M_G$-length list of features

1 $\mathbf{S} \leftarrow \mathbf{S}_D \cap \mathbf{S}_G$ // intersection of 2 feature lists
2 **for** $i \leftarrow 1$ **to** $N$ **do**
3 $\quad \bar{\mathbf{S}}, \mathbf{V}, \mathbf{E} \leftarrow [], [], []$ // initialize list of features, node vectors and edge indices
4 $\quad \mathbf{A}_e \leftarrow []$ // optional edge attributes
$\quad$ // Construct list of eligible features and node vectors
5 $\quad$ **foreach** $\mathrm{s} \in \mathbf{S}$ **do**
6 $\quad\quad v \leftarrow \mathbf{X}_D(i, \mathbf{S}_D.\mathrm{index(s)})$
7 $\quad\quad$ **if** *isNodeEligible(v)* **then** // node value passes eligibility check
8 $\quad\quad\quad \bar{\mathbf{S}}.\mathrm{append(s)}$
9 $\quad\quad\quad \mathbf{v}_x \leftarrow \mathrm{nodeFeat}(v, \mathrm{s}, \text{**kwargs})$ // construct node feature from v and other info
10 $\quad\quad\quad \mathbf{V}.\mathrm{append}(\mathbf{v}_x)$
$\quad$ // construct edge indices
11 $\quad$ **foreach** $\mathrm{s}_1 \in \bar{\mathbf{S}}$ **do**
12 $\quad\quad$ **foreach** $\mathrm{s}_2 \in \bar{\mathbf{S}} \setminus \mathrm{s}_1$ **do**
13 $\quad\quad\quad$ **if** *isEdgeEligible*$(\mathcal{G}, s_1, s_2)$ **then** // edge exists in KB
14 $\quad\quad\quad\quad e_1, e_2 \leftarrow \bar{\mathbf{S}}.\mathrm{index}(s_1), \bar{\mathbf{S}}.\mathrm{index}(s_2)$
15 $\quad\quad\quad\quad \mathbf{E}.\mathrm{append}((e_1, e_2))$
16 $\quad\quad\quad\quad \mathbf{a}_e \leftarrow \mathrm{getEdgeAttribute}(\mathcal{G}, \mathrm{s}_1, \mathrm{s}_2)$
17 $\quad\quad\quad\quad \mathbf{A}_e.\mathrm{append}(\mathbf{a}_e)$
18 $\quad \mathbf{G}_i \leftarrow \{\mathbf{V}, \mathbf{E}[, \mathbf{A}_e]\}$ // output graph is formed by $\mathbf{V}, \mathbf{E}$ and (optional) $\mathbf{A}_e$

The methods described herein may be performed by a module for executing algorithm 1. The module may load, or take as an input, a table $\mathcal{D}$. The table may be a biological dataset. A knowledge base $\mathcal{G}$ may also be loaded/inputted. Alternatively, the knowledge base may exist for example in the cloud. In either instance the method may comprise accessing the knowledge base.

The table $\mathcal{D}$ may have a size of N rows and $M_0$ columns, and in this example is represented by a matrix $X_D$, and the list of column names (i.e. feature labels) is $S_D$. $X_D(i, j)$ is the value of table cell at row i and column j. The KB $\mathcal{G}$ may be represented by a matrix $X_G$ and the list of features (feature labels) $S_G$. The module may be configured to output N graphs $\{G_i\}$ where $G_i$ is transformed from row i in table $\mathcal{D}$ (i=1,..., N). In some examples the module may output few than N graphs. That is, a graph may not necessarily be constructed for every row/sample in the table.

Given a biological tabular dataset D with N rows and $M_0$ columns, together with targets $Y_D \in \mathbb{R}^N$ (i.e., classifications of conditions), the steps of the methods described herein, may train a machine learning model $f_\theta : x_i \rightarrow y_i$, where sample $x_i$ is row i-th in D. It may be assumed that, if two features are related according to a certain knowledge base, their corresponding nodes (which may otherwise be referred to as neurons) are connected during the modelling process.

The transformation steps for converting/transforming a tabular dataset to a graph may be as follows:

i.) Obtain a joint list of features $S$ by intersecting the table column names $S_D$ with the KB features list $S_G$. This may serve as the candidate feature list across all samples (L1 in Algorithm 1).

The tabular dataset D may be characterized by a list of features (column names) $\boldsymbol{S}_D$ and the value matrix $\boldsymbol{X}_D \in R^{N \times M_0}$. A knowledge base $\mathcal{G}$ may include a list of features $\boldsymbol{S}_G$ and comprise a pairwise relation matrix $\boldsymbol{X}_G \in R^{G \times G}$, where $\boldsymbol{X}_G(s_i, s_j)$ represents the relation between two features $s_i, s_j \in \boldsymbol{S}_G$. $\boldsymbol{X}_G$ may represent a binary square matrix for each knowledge base. The list of features common in both the dataset $\mathcal{D}$ and knowledge base $\mathcal{G}$, may be defined as $\boldsymbol{S} = \boldsymbol{S}_D \cap \boldsymbol{S}_G$, and the remaining features may be dropped. That is, features present in both the biological dataset and the knowledge base may be used to construct the graphs and for example any features not contained within the knowledge base may be

excluded from the graphs.

Next, iterate through all rows in table $\mathcal{D}$ . The following steps are for a row *i.*
ii.) Construct a list of eligible features $\overline{\boldsymbol{S}} \in \boldsymbol{S}$ (L3-8). A feature s may be deemed eligible if the cell value (attribute) at row i and column name (feature label) s passes an eligibility check defined by a function *isNodeEligible().* By default, *isNodeEligible()* may be configured to always return True, meaning $\overline{\boldsymbol{S}} = \boldsymbol{S}\ \forall i \in [1, N]$ and all output graphs may therefore have the same number of nodes. This may enforce the same inductive bias through, for example, message passing when being modelled with GNNs.

However, domain knowledge may be applied to filter/remove certain features based on their attributes. For example, for cancer subtyping prediction in CNV data, it may be assumed that only genes (features) having variants (attributes) with value != 0 (i.e., not equal to zero) are of interest, therefore *isNodeEligible(v)* may be configured to return False if v equals 0 otherwise True (more detail of this example is provided in relation to Figure 7). This step may be referred to as node pruning and may simplify the graphs, diversify the edge structure, and lead to improved performance.

iii.) Construct the corresponding node vectors V (L9-10). Each row $x_i$ may be converted to a to a graph $G_i$ = {V, E} where V is the vertices and E is the edges. For each feature $s \in \overline{\boldsymbol{S}}$, a node may be created for the graph with representation vector $\boldsymbol{v}_x$ defined by function *nodeFeat().* By default, $\boldsymbol{v}_x$ may have the cell value (attribute) at row i and column named (feature label) s in table D (denoted v in Algorithm 1). However, other information such as the index of feature s in a global candidate list S may be integrated into the node. For instance, suppose **S** = {s1, s2, s3, s4,..., s100}. Within row i, derive a subset of eligible features $\overline{\boldsymbol{S}}$= {s2, s3, s5...,s100} that pass the *isNodeEligible()* check. The "other information" here may refer to the index of each element in $\overline{\boldsymbol{S}}$ with respect to **S**. Specifically, for s2, the index of s2 in S is 2 (assuming the index starts from 1). For s3, the index of s3 in S is 3. For s5, the index of s5 in S is 5. In this case, method *nodeFeat()* may be defined as:

**Algorithm 2:** nodeFeat() definition.

**Input:** cell value $v$, feature $s$, global candidate list $S$
**Output:** Node vector $v_x \in \mathbb{R}^2$

```
1 v_x ← [v, S.index(s)]
2 return v_x
```

An example of a node construction is provided in Figure 4. To build the node vectors, each cell at column $s_j \in \overline{\boldsymbol{S}}$ of row $\boldsymbol{x}_i$ may be converted to a node with associated representation vector $\boldsymbol{v}_x = [\boldsymbol{X}_D(i,j),\ j'] \in \mathbb{R}^2$ where $j'$ is the index of $s_j$ in $\boldsymbol{S}$. While the vector is represented in the order of cell value followed by index, the order of the vector may be reversed, as long as it is used consistently across the samples. The feature indices may be encoded to ensure that the output graph is permutation invariant. That is, the encoding may ensure that the mapping between table row $\boldsymbol{x}_i$ (otherwise referred to as *i*) and output graph $\boldsymbol{G}_i$ is bidirectional (i.e., $\boldsymbol{x}_i$ may be reconstructed from $\boldsymbol{G}_i$ regardless of view into the graph).

iv.) Construct the edge indices E (L11-15) and optional edge attribute $A_e$ (L16-17). For every pair of features ($s_1$, $s_2$) within $\overline{\boldsymbol{S}}$, if the connection between $s_1$ and $s_2$ exists in the KB, then the corresponding indices of $s_1$ and $s_2$ in $\overline{\boldsymbol{S}}$ may be added to E. The connection between $s_1$ and $s_2$ may be defined by the function *isEdgeEligible()* in the algorithm 3, where for example, for the MoNDO KB in CNV cancer subtyping task:

**Algorithm 3:** isEdgeEligible() definition example for CNV data.

**Input:** KB $\mathcal{G} = \{X_G, S_G\}$, feature $s_1$, feature $s_2$
**Output:** Boolean - connection between $s_1$ and $s_2$

```
1 j1, j2 ← S_G.index(s1), S_G.index(s2)
2 if X_G(j1, j2) > 0 then
3 |  return True
4 else
5 |  return False
```

The edge matrix E may be constructed directly from the knowledge base, using for example the equation:

$$E = \{(s_i, s_j)\}\ \forall\ s_i, s_j\ \in\ S\ if\ X_G(s_i, s_j)! = 0\ and\ i\ ! = j \quad (1)$$

The direction of the edges may depend on the nature of the feature relationship in the knowledge base. If the KB contains more information between two features besides their connection, this information (extracted for example via the

function getEdgeAttribute()) may serve as an edge attribute, stored in $A_e$.

Thus, the graph $G_i$ for row i may include node vectors V, edge indices E and optionally edge attributes $A_e$ (L15).

**Figure** 4 shows an example node construction 400. In particular, the Figure shows an example node vector projection for converting a node 410 to an embedding node 420 suitable for a GNN.

As an example, a sample may comprise gene identifications (IDs) 425 as features (or specifically here feature index) and each gene ID may be assigned a Copy Number Variants 427 as an attribute. A gene ID here refers to the index of the gene in the gene list S. For example, BRCA1 may be the gene name (or feature label), and it may have the feature index 1234 in the gene list S, therefore its gene ID is 1234. A node in a graph may be constructed with the gene ID and CNV. To configure the node for inputting into a GNN, the node feature and attribute may be projected to an embedding space. That is, a node vectors $\boldsymbol{v}_x$ may be projected to an embedding space P of dimension n. In this example, the node vectors may be projected to n-dimensional vectors, as shown in Figure 4. That is, the feature label may be converted into a feature label vector within the embedding space and the attribute may be converted into an attribute vector within another embedding space.

The first element (feature index) of $\boldsymbol{v}_x$ may be transformed with an embedding layer $\mathcal{P}_0$ and the second element (tabular cell value) may be transformed with a linear layer $\mathcal{P}_1$, each with dimension *n*/2 (i.e. the same dimension):

$$P = [\mathcal{P}_0(\mathbf{V}_0), \mathcal{P}_1(\mathbf{V}_1)]_{\theta_0} \quad (2)$$

where [, ] represents a concatenation operation and $\theta_0$ represents a learnable parameters of $\mathcal{P}_0$ , $\mathcal{P}_1$. An Embedding layer projects a discrete input value to a representation vector, while a Linear layer projects a floating point input value to a representation vector. Here, an embedding layer was used to encode the feature index (hence, discrete integer) and a linear layer was used to encode the cell value (floating point). These layers may form the first components of a Deep Neural Network (DNN).

Considering the example with CNV values, the CNV value part of the node vector may be encoded using the Linear layer. The CNV value may be encoded using a linear layer as the CNV value may be a numerical value. For instance, CNV values may be one of {-2,-1,0,1,2} which are discrete, however they may be treated as floating point values similarly to RNA and clinical data. The inventors treated the tabular cell values as floating point data and used a Linear layer to encode them.

The gene index may be encoded using an Embedding layer. The feature labels (names) (or column names) are usually text. The features labels may be converted to a set of indices which are discrete integers then an embedding layer maybe applied to encode them. For example, a feature set {s1, s2, s3, s4} may be converted to the feature indices { 1, 2, 3, 4}. The encoded outputs of the feature and attribute may be concatenated to form the n-dimension vector.

**Figure 5** shows an example structure 500 of a graph neural network (GNN) which may be used with the methods described herein.

A graph 510 may be constructed from a knowledge base. The nodes of the graph may be constructed as described in Figure 4 for being input into a GNN model. The GNN model may be trained for the task of interest, in this example classification, using a graph set $\{G_i\}$ for the KB (see Figure 3).

The GNN model may consist of a set of processing blocks 520, 530. Each block may comprise a GNN parsing layer 522 ,532 followed by an activation layer 525, 535 and/or a normalization layer (not shown). The GNN parsing layer may aggregate information (i.e., feature embeddings) from a node's neighbours and update the nodes representation by combining the aggregated information with the node's current state. This may enable the model to capture the structural and relational information of the graph while learning node or graph-level features. The parsing layers may be at least one of a Graph Convolutional Network (GCN) Layer, a GraphSAGE (SAGEConv) Layer and/or a Graph Attention Network (GAT) Layer. The parsing layers may be task-dependent and may be determined through parameter tuning. The GNN model may be trained by backpropagating a gradient from a prediction output to the graph input.

The graph may be input into a first parsing layer 522 which may aggregate the features of the node (e.g., in an example the Gene ID embedding and CNV embedding) with the features of the direct neighbours of the node (direct neighbours being neighbouring nodes connected with the node by an edge). The features may be aggregated using, for example a sum of average of the features. The parsing layer may also comprise a linear layer which applies a weighted matrix to transform the aggregated layer. The weighted matrix may comprise learnable weights which are updated during training of the GNN.

The parsing layer may comprise an activation layer, such as ReLU activation. The activation layer may introduce an element of non-linearity. This process may be repeated for each node within the input graph.

The parsing layer may comprise a normalization layer. The normalization layer may ensure that the graphs (vector representations of the graphs) remain well-scaled and may prevents certain numerical issues such as exploding or vanishing gradients. Furthermore, the normalization layer may stabilize the propagation of information across graph

structures. The first parsing layer may output an updated representation of the graph.

The updated representation may be input into a second parsing layer 532. The second parsing layer may comprise the same modules as the first parsing layer (e.g., aggregation layer, linear layer, activation layer and/or normalization layer). The second parsing layer may perform substantially the same process as the first parsing layer but may also allow neighbours of neighbours (i.e., second hop neighbouring nodes) to be included in the aggregation step. The GNN model may comprise further parsing layers up to, for example, a limit in which all hops between neighbours are considered.

Data augmentation may be performed during the GNN model training. An example of data augmentation may be randomly dropping nodes and/or edges of the training samples (i.e., the graphs). The data augmentation may be performed assuming a sample still retains meaningful signals for the training task even if it is slightly corrupted (by losing some nodes and/or edges).

The final parsing layer may output a node embedding 540. The node embedding may comprise learned representations of each node in the graph after being passed through the message parsing layers. The node embedding may represent learned features for each node and each nodes neighbours and edges.

To generate a classification for the graph, the node embedding may be input into a readout layer 550. The readout layer, which may otherwise be referred to as a pooling layer, may combine the features of the nodes into a readout embedding (a vector). The readout layer may combine the node embeddings using, for instance, mean pooling, sum pooling, max pooling and/or attention-based pooling. Of course, other pooling methods may be used.

The output of the readout layer may be input into a classification layer 560. The classification layer may comprise a probability function for predicting the classification probability of the graph (that is, the probability of the graph being classified as each of the possible classification labels 570). In this example, 4 possible classifications exist. An example function which may be used for classification is a softmax function. During training of the GNN, before being intput into the classification layer, the output of the readout layer may first be input into a dropout layer (not shown). The output of the dropout layer may then be input into the classification layer.

The classification layer may further comprise an MLP layer before the probability function is applied. The MLP layer may improve the classification accuracy by allowing non-linear decision boundaries to be learned by the GNN model. The classification layer may include one MLP layer after the Read Out layer to map the d-dimensional vector to the N-way logits (N=4 classes in the breast cancer subtyping problem discussed below). The MLP may comprise learnable weights which are applied to the output of the readout layer.

The GNN model may be trained using backpropagation and adjusting each learnable weight in the embedding and/or parsing (and if used, MLP) layers. The exact architecture of the GNN model and other hyperparameters may be determined through parameter tuning. The GNN may be trained in a supervised learning manner. That is each graph (with each graph being constructed from a sample) may be associated with a correct classification and the GNN may be trained in a supervised learning manner by updating weights based on a comparison of the predicted classification and the correct classification for that graph.

While the graph in the Figure is shown graphically, the graph may be represented in a matrix form for input into the GNN. For example, the graph may be represented by an adjacency matric, denoted by A. For a graph with N nodes, the dimension of A may be ($N \times N$), with each entry in the matrix indicating a relationship between nodes.

As shown in Figure 4, each node may comprise a feature including a feature label, and an attribute. If a node comprises multiple elements such as the feature label and the attribute, a node feature matrix F may be created with dimensions ($N \times f$), where f is number of elements in the nodes). Thus, each row in F may correspond to a node (i.e. a feature) and each column may correspond to an attribute. Thus, the adjacency matrix may indicate connections/relationships between nodes and the node feature matrix may captures the characteristics of each node in the graph. The above example is provided for a single graph. However, graphs, forming a graph set, may be input into the GNN to train the GNN.

**Figure 6** shows another block diagram 600 for of an architecture for classifying a medical condition of samples using a dataset. The block diagram shown in the Figure is substantially the same as the block diagram in Figure 2, however the block diagram in Figure 6 extends the classification method for use with multiple knowledge bases (KB1, KB2, ... KB k).

Different knowledge bases may comprise relationships between features for different areas. For instance, one knowledge base may comprise relationships related to the same disease, another may comprise relationships related to the same pathway and another may comprise relationships related to molecular interactions.

For each knowledge base, a graph set may be constructed according to the methods described herein. That is, if the number of KBs is greater than 1, multiple graph sets may be constructed, one for each KB. A graph may be constructed for one or more samples in the dataset to form a graph set. A GNN for each KB may be trained using the graph set. Thus, for multiple KBs, multiple GNN models may be trained. Given a sample, each GNN model may outputs its own prediction scores (that is, if used in a classification task the GNN may output its own predicted classification of a medical condition). A fusion model 610 may be trained to combine the prediction scores into a final (global) prediction result (denoted as prediction 620 in the Figure).

The fusion model may comprise a (light weight) linear matrix $W$ of size K by C, where K is number of KBs and C is

the dimension of the prediction score vector (i.e., the number of possible classifications). The fusion model may be trained using a similar method as the GNN model training described above (e.g., via backpropagation of a loss of an object function that minimizes the difference between the prediction and ground truth label).

Considering an example with 3 separate KBs and 4 classification possibilities for, e.g., CNV and RNA data, a late fusion approach may be applied to combine the predictions from each model trained for each KB. The fusion model may comprise a single matrix $W \in \mathbb{R}^{3\times4}$ that produces a weighted sum over outputs $o^i \in \mathbb{R}^4$ $i$ = 1, 2, 3 of the $i$-th component models:

$$\bar{o}_j = \sum_i w_{ij} * o_j^{(i)} \quad (3)$$

W may be a trainable variable which may be trained using gradient descent.

In summary, Figure 6 shows a modelling pipeline which may be used for biological tabular data. Given K Knowledge Bases (K>=1), corresponding K graph sets may be constructed. Then, K GNN models may be trained separately, one model for each graph set. Finally, a fusion model may be trained to incorporate K predictions from these separate models into a final prediction.

**Figure 7, 8, 9a and 9b** provide worked examples for implementing the methods described herein. In the below examples, the inventors studied Breast Invasive Carcinoma (TCGA BRCA) - a comprehensive collection of genomic and clinical data for breast cancer patients. TCGA BRCA contains 3 biological tabular datasets ~(BioTDs) - Copy Number Variation (CNV), RNA-sequence (RNA-seq, henceforth RNA) and Electrical Health Records (Clinical). These datasets can be download from cBioportal *[*E. Cerami, J. Gao, U. Dogrusoz, B. E. Gross, S. O. Sumer, B. A. Aksoy, A. Jacobsen, C. J. Byrne, M. L. Heuer, E. Larsson et al., The cbio cancer genomics portal: an open platform for exploring multidimensional cancer genomics data, Cancer discovery 2, 401 (2012*).].*

In the datasets, CNV quantifies duplications and deletions in DNA segments. RNA provides a transcriptomic profile that captures gene expression levels in a tissue sample. Clinical encompasses patient history, lab results, demographics, and other clinical information.

The target classes in the dataset were 4 cancer subtypes and were highly unbalanced: Luminal A (53.5%), Luminal B (20.6%), Her2-enriched (7.8%) and Basal-like (18.1%) per PAM50 settings *[*S. Parker, M. Mullins, M. C. Cheang, S. Leung, D. Voduc, T. Vickery, S. Davies, C. Fauron, X. He, Z. Hu et al., Supervised risk predictor of breast cancer based on intrinsic subtypes, Journal of clinical oncology 27, p. 1160 (2009*)., and* D. Netanely, A. Avraham, A. Ben-Baruch, E. Evron and R. Shamir, Expression and methylation patterns partition luminal-a breast tumors into distinct prognostic subgroups, Breast Cancer Research 18, 1 (2016*).].* Only patients who had all CNV, RNA and Clinical data were retained. In this instance, 977 patients were retained. This enabled direct comparison with an MLP baseline *[*Anonymous Authors. Scalable and loosely-coupled multimodal deep learning for breast cancer subtyping, 2025. Under review*]* whose authors constructed the train/validation/test dataset this way, *see also European patent application:* EP24191385.4. The MLP results in Table 3, 5 and 6 herein are taken directly from the MLP paper. The same dataset configuration was therefore used to allow for a direct comparison with the MLP's paper. However, it is not a requirement that only samples which appear in all three datasets are kept and the 3 benchmarks of CNV, RNA and Clinical were treated separately by the inventors.

While the CNV and RNA data were numerical, the Clinical data was both numerical (e.g. age at diagnosis), ordinal (e.g. ImmunoHistoChemistry IHC scores), and categorical (e.g. International Classification of Diseases, ICD-10 codes). The categorical and ordinal features were converted to one-hot encoding and the Clinical data was standardized via z-scoring.

The number of columns in the CNV, RNA and Clinical BioTDs were 23286, 20530 and 138, respectively. Unlike existing tabular datasets with a medium size (n >3000) and medium-to-small columns (d < 500), the CNV and the RNA datasets had much smaller n/d ratio (< 5%), which the inventors found illustrates a more realistic scenario of a biological tabular data and introduces challenges for modelling techniques. The inventors also used Clinical data for increased diversity in data type (phenotype versus omics) and values (heterogeneity).

In the below examples four knowledge bases were used. For CNV and RNA data, three popular public data sources - MoNDO [Mungall et al. The monarch initiative: an integrative data and analytic platform connecting phenotypes to genotypes across species. Nucleic acids research, 45(D1):D712-D722, 2017*],* Reactome [Fabregat et al. The reactome pathway knowledgebase. Nucleic acids research, 46(D1):D649-D655, 2018.*]* and BioGrid *[*Oughtred et al. The biogrid interaction database: 2019 update. Nucleic acids research, 47(D1):D529-D541, 2019.*]* were accessed. Gene relation networks were constructed using the KBs. Within each network, nodes may represent genes (or their protein products), while edges may connect genes to each other based on their relationships/associations as reflected in that data source.

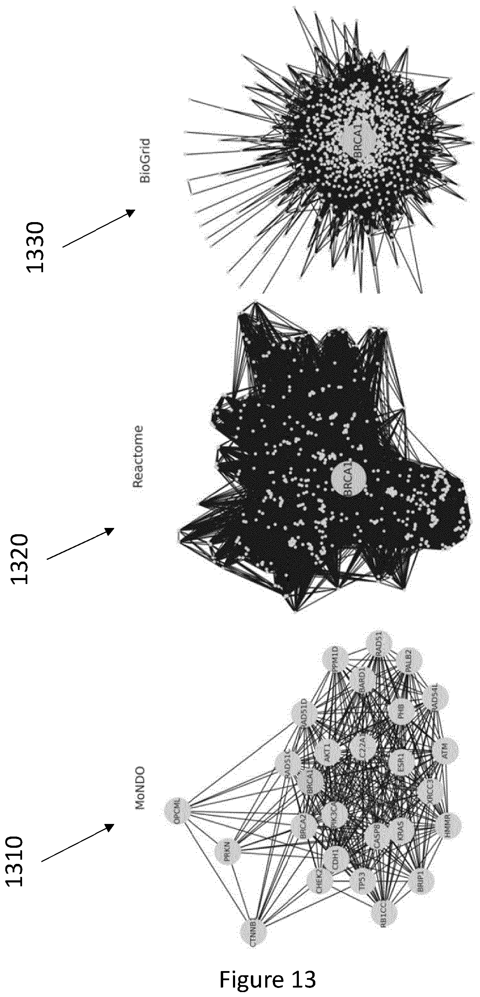

The MoNDO knowledge base connects two genes if genome-wide association studies (GWAS) report them both to be associated with the same disease. Reactome connects two genes if they are annotated to the same biological

pathway group, while BioGrid relies on physical or genetic (molecular) association between them. The knowledge bases are visualised as graphs in Figure 13.

The inventors found that for clinical data, there may not be a complete KB defining clinical feature relationships. The inventors manually constructed a KB by utilizing previously published research, medical textbooks, and other verified medical resources. The knowledge base may be constructed automatically by pooling information. The knowledge base constructed by the inventors comprised nodes which signified clinical features, while edges connected these features if there was evidence in the literature that they influence each other. For example, feature 'HER2 IHC score' (human epidermal growth factor receptor 2 Immunohistochemistry score) and 'HER2 FISH status' (HER2 fluorescence in situ hybridization) are strongly correlated according to [Daniela Furrer, Simon Jacob, Chantal Caron, Francois Sanschagrin, Louise Provencher, and Caroline Diorio. Concordance of her2 immunohistochemistry and fluorescence in situ hybridization using tissue microarray in breast cancer. Anticancer Research, 37(6):3323-3329, 2017*]*. The clinical KB developed by the inventors was not exhaustive, as some nodes and edges may be missing or are not evidenced. This may pose a practical challenge, however the inventors still found the models to function effectively.

Overall, the constructed MoNDO KB had 4152 nodes and 54690 edges, excluding self-loops. The numbers of nodes/edges for the Reactome, Biogrid and Clinical KBs were 11716/1937505, 18763/932080 and 50/224, respectively.

In the three examples below (CNV, RNA and Clinical), 10-fold cross-validation was applied at a train-validation-test of 0.8-0.1-0.1, and performance metrics were averaged across all folds. To address the unbalanced issue for the 4 classifications of the cancer, the training data was oversampled to match the size of the majority class. This was applied to the methods described herein and all baselines for a fair comparison.

Graphs sets were constructed on all 3 datasets as described below. Standard accuracy (micro), Area Under Curve (macro AUC), F1 score (macro) and Cohen Kappa (KC) were used to indicate the performance of the methods disclosed herein compared with baselines. Among these, AUC and F1 scores were measured for each class and averaged at the end, therefore accounting for class imbalance in the test set. KC is a statistical metric widely used to measure the agreement between the predicted and ground truth labels, taking into account the agreement occurring by chance. All metrics had an upper threshold value of 1, where generally the higher the better.

The models used to implement the methods described below were trained using the PyTorch library. For CNV and RNA data, each model was trained for up to 200 epochs, while Clinical models were trained for 400 epochs, with early stopping applied when validation performance ceased to improve. The Adam optimizer was used with an initial learning rate of $10^{-3}$ for CNV and RNA, and $10^{-4}$ for Clinical data, utilizing a cosine annealing schedule. The batch size was set to 64 for CNV and RNA, and for each training input graph, 6400 nodes and 3200 edges were randomly selected for data augmentation. Conversely, for Clinical data, the batch size was set to 16, and no random selection of nodes or edges was conducted.

A hyperparameter search was performed with up to 50 random search iterations for the methods described herein and the baselines. The hyperparameter search may comprise a grid search, random search, TPESample, and other suitable search methods. The inventors used a sequential model-based optimization method implemented by the Python library Optuna. This method starts as a random search, but after each evaluation it analyses the results and uses this information to guide future parameter selections . Examples of parameters which were searched include model architecture parameters such as: number of Layers of the GNN, hidden dimension (the size of the node embedding), aggregation function, activation function, dropout rate, batch normalization and residual connections, and/or training hyperparameters such as: learning rate, optimizer type (e.g., Adam), loss function, weight decay and gradient clipping. Other parameters may also be considered such as graph sampling parameters (e.g., batch size) and training strategy parameters (e.g., number of epochs).

The inventors used a standard Intel Xeon 2.3GHz CPU server with 128GB RAM and a single 48GB Nvidia Quadro RTX8000 GPU. It took approximately 3 hours to complete a 10-fold cross-validation for the methods described herein. Of course, a different GPU//CPU/other processing units may be used for training. Table 1 below shows the selection of parameters for each of the three examples. More detail for each example is provide below for each example.

*TABLE 1 Details of model architecture for each dataset and knowledge base.*

| | CNV | | | RNA | | | Clinical |
|---|---|---|---|---|---|---|---|
| | MoNDO | Reactome | Biogrid | MoNDO | Reactome | Biogrid | |
| Feature dimension (*d*) | 128 | 128 | 512 | 512 | 1024 | 256 | 32 |
| Architecture | SG | GEN | GCN | GCN | SAGE | GAT | PNA |
| Number of layers | 2 | 2 | 4 | 2 | 2 | 2 | 3 |
| Activation | GELU | PReLU | GELU | GELU | ReLU | GELU | ReLU |
| Normalization | - | Layer | Batch | Instance | Instance | Instance | Batch |

The methods were compared with 2 deep learning - MLP [Anonymous Authors. Scalable and loosely-coupled multimodal deep learning for breast cancer subtyping, 2025. Under review.*], [*Xingze Wang, Guoxian Yu, Zhongmin Yan, Lin Wan, Wei Wang, and Lizhen Cui. Lung cancer subtype diagnosis by fusing image-genomics data and hybrid deep networks. IEEE/ACM Trans. Comp. biology and bioinformatics, 20(1):512-523, 2021], [T Liu, J Huang, T Liao, R Pu, S Liu, and Y Peng. A hybrid deep learning model for predicting molecular subtypes of human breastcancer using multimodal data. Irbm, 43(1):62-74, 2022] and TabPFN [Noah Hollmann, Samuel Muller, Katharina Eggensperger, and Frank Hutter. Tabpfn: A transformer that solves small tabular classification problems in a second. arXiv preprint arXiv:2207.01848, 2022*],* and 3 classical baselines - XGBoost [Tianqi Chen and Carlos Guestrin. Xgboost: A scalable tree boosting system. In Proc. ACM SIGKDD, pages 785-794, 2016*],* LASSO [Robert Tibshirani. Regression shrinkage and selection via the lasso. Royal Stat. Society, 58(1):267-288, 1996*]* and RIDGE [Donald W Marquardt and Ronald D Snee. Ridge regression in practice. The American Statistician, 29(1):3-20, 1975*.].* MLP was benchmarked for its popularity in the biomedical domain, while the rest of the baselines were selected as they are designed to work on small/medium tabular datasets.

**Figure 7** shows an extract from the CNV dataset 700 which may be used with the methods described herein. In particular, the methods may be used for breast cancer subtyping classification using the CNV dataset. In this instance the breast cancer may be the medical condition being classified. The dataset comprises a first column with patient ID 705, with each patient ID referring to a different sample. Each sample comprises columns with feature names 710 (i.e., features and in particular feature labels, in this instance gene labels) and feature values 715 (i.e., attributes, in this instance CNV scores).

The classification targets (denoted as target value 720 in the Figure) were as above with the 4 classes: 0 (Luminal-A), 1 (Luminal-B), 2 (Her2-enriched) and 3 (Basal-like). Each sample contains a record of a patient.

The CNV dataset had 23286 columns (excluding the patient ID column which was not modelled). Each column represents a gene. The cell value represents number of copies of gene segment that is different from a reference genome. This data is homogeneous and has 5 possible values: -2 (deep deletion), -1 (shallow deletion), 0 (diploid/no difference), +1 (gain) and +2 (amplification).

The MoNDO KB contained information of 3915 genes overlapping with the genes in the CNV dataset in this example. Two genes are connected if related to the same disease. Reactome shared 10393 genes with the CNV data. Two genes are connected if related to the same pathway. Biogrid shared 17735 genes with the CNV data. Two genes are connected if they have a molecular interaction.

The connection between any two genes in the 3 KBs may be binary, for example either 0 (no edge) or 1 (has edge). Given the number of overlaps of the KBs and the CNV dataset, the KB connection matrices may be defined as $X_G \in \{0,1\}^{3915\times3915}$ for MoNDO, $X_G \in \{0,1\}^{10393\times10393}$ for Reactome and $X_G \in \{0,1\}^{17735\times17735}$ for BioGrid.

Edge attributes $A_e$ were not available/supported in this example. However, examples of edge attributes may include: a gene A and gene B may be related since they are both the biomarker of medical condition/disease X (e.g. a cancer or subtype of cancer). X may be included as the edge attribute for the edge connecting A and B. Another example of an edge attributes may be from the knowledge base providing information about the relation strength between two genes. This relation strength may be included in the edge attribute. The following paper shows how the distance between 2 groups of genes may be measured: Núñez-Carpintero, Iker, et al. "The multilayer community structure of medulloblastoma. "Iscience 24.4 (2021*).*

It was assumed that if a gene has a CNV value of zero (diploid), it is not meaningful for the cancer subtyping task. The node eligibility function was therefore defined as follow using algorithm 4:

**Algorithm 4:** isNodeEligible() definition for CNV data.

**Input:** cell value $v$

**Output:** Boolean - is this cell eligible to become a graph node

```
1 if v! = 0 then
2 |   return True
3 else
4 |   return False
```

That is, if a feature label had an attribute with numerical value zero, that feature label was not included in the graph. Examples definitions of nodeFeat() and isEdgeEligible() for all KBs are shown in Algorithms 2 and 3 respectively.

To construct the GNN models, the node vectors were first projected to an n-dimensional vector, as shown in Figure 4 and described above. The CNV value part of the node vector was encoded using a linear layer to generate an attribute vector while the gene index was encoded using an embedding layer to generate a feature label vector, each with dimension n/2. The outputs were concatenated to form a n-dimension vector.

The value of the dimension n and model architecture for each KB for the CNV dataset is shown in Table 2 below. Each block in the GNN model may consist of a GNN parsing layer followed by a normalisation layer and an activation layer

(the order of the layers vary). A readout layer may be applied on the final output, followed by a dropout layer and a 4-way classifier. Of course, other suitable GNN structures may be used with the method described herein. For instance, the GNN may not include the dropout layer. Further, the dropout layer may be implemented during training but may be removed during the deployment phase. It is a common practice to use Dropout only during training to reduce overfitting.

Table 2 Details of GNN model architecture to model CNV data.

| | MoNDO | Reactome | BioGrid |
|---|---|---|---|
| Message passing algorithm | SGConv | GENConv | GCNConv |
| # hidden dim. (n) | 128 | 128 | 512 |
| # blocks | 2 | 2 | 4 |
| Activation | GELU | PReLU | GELU |
| Normalisation | none | LayerNorm | BatchNorm |
| Dropout | 0.25 | 0.3 | 0.1 |
| Readout | Max Pool | Max Pool | Max Pool |

Table 2 shows that the architecture for each KB may vary. The architecture/ parameters for each KB may be determined through parameter tuning. A hyperparameter search was performed with up to 50 random search iterations and the best performing parameters and architecture were selected.

Three separate GNN models were trained, one for each KB. Once the GNN models were trained, a fusion model was trained to combine the predictions of the three models. As there were 3 GNNs and 4 classes for the prediction, a fusion weight matrix of size 3x4 was generated. That is, the fusion weight matrix size may comprise the number of GNNs models the number of classes.

All models were trained using batch size of 64, with the Adam optimizer, and an initial learning rate 0.001 on the first 30 epochs, which was then reduced by 10x after. Different training parameters may be set according to, for example, parameter tuning.

For the MoNDO and Reactome models, for each training sample in a minibatch, a maximum of 6400 random edges were retained and the rest of the edges were dropped to fit into memory and diversify the training data. As the BioGrid KB comprises a large number of shared nodes with the CNV dataset, a maximum of 6400 nodes and 3200 edges were retained during training. The inventors found that if memory is not an issue, keeping all nodes and edges may lead to improved performance. At test time, no augmentation was performed, and the batch size was reduced to 2 to fit the GPU memory.

Table 3 shows performance scores for the methods described herein, both for individual classifications for single KBs and for combined classifications using the fusion model, along baselines. While the KBs only covered part of the genes available in the CNV datasets, the fusion model outperformed the baselines on all metrics.

*Table 3 Performance of GNN model trained with CNV dataset versus baselines.*

| Method | Accuracy | AUC | FI | $K_C$ |
|---|---|---|---|---|
| X2Graph (fusion) | **0.7410** | **0.8935** | **0.6858** | **0.5892** |
| X2Graph Reactome | 0.7321 | 0.8796 | 0.6815 | 0.5776 |
| X2Graph MoNDO | 0.7239 | 0.8892 | 0.6561 | 0.5635 |
| X2Graph BioGrid | 0.7143 | 0.8855 | 0.6303 | 0.5421 |
| TabPFN | 0.7213 | 0.8630 | 0.5875 | 0.5414 |
| MLP | 0.7025 | 0.8284 | 0.6126 | 0.5283 |
| LASSO | 0.6952 | 0.8344 | 0.6391 | 0.5107 |
| XGBoost | 0.7312 | 0.8831 | 0.6837 | 0.5738 |
| Ridge | 0.6291 | 0.6720 | 0.5239 | 0.3815 |

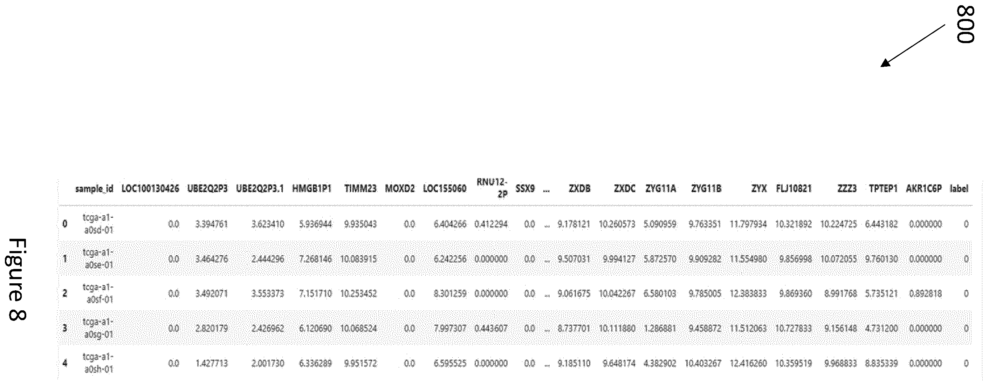

**Figure 8** shows an extract from the RNA-sequence dataset 800 which may be used with the methods described herein. In this example the dataset was substantially the same as the CNV dataset described in relation to Figure 7 above, except that it had 20530 columns and the cell values were floating point numbers. The remaining columns remained the same (e.g., patient ID, feature name, and target value). To reduce the value range of the feature values, a logarithm was

applied: x:=log(1+x). Figure 8 shows an extract of the dataset of the table after this logarithm transform.

The same 3 KBs were applied as before. The number of common genes between each KB and the RNA data were 3584 genes for MoNDO, 9918 genes for Reactome and 16646 genes for BioGrid. In this example there were no other assumptions so isNodeEligible() was set to always return True, whilst nodeFeat() and isEdgeEligible() were the same as the CNV modelling.

A data augmentation step was applied to the graphs before being input into the GNN for training. As described above, the performance of the model may be improved when no nodes are dropped by design. RNA graphs are generally larger than CNV graphs so to fit on the same GPU under the same settings, more nodes and edges were dropped during training in the form of data augmentation. Specifically, for MoNDO, all nodes were kept and a max edge number is 6400 was retained for each training sample. For Reactome and Biogrid, the maximum number of nodes and edges for each training sample were 6400 and 3200 respectively. Of course, the number of nodes and edges kept during training may depend on the capacity of the architecture used for training. If the architecture, e.g., a GPU and memory, is capable of accepting larger dataset, then fewer nodes and edges may be dropped. Table 4 shows the model architecture details for this example.The remaining settings for the GNN models were retained and were the same as CNV example. Results are show in Table 5.

*Tabel 4 Model architecture for each KB for RNA dataset.*

| | MoNDO | Reactome | BioGrid |
|---|---|---|---|
| Message passing algorithm | GCNConv | SAGEConv | GATConv |
| # hidden dim. (n) | 512 | 1024 | 256 |
| # blocks | 2 | 3 | 2 |
| Activation | GELU | SELU | GELU |
| Normalisation | InstanceNorm | LayerNorm | InstanceNorm |
| Dropout | 0.15 | 0.35 | 0.1 |
| Readout | Max Pool | Mean Pool | Max Pool |

*Table 5 Performance of GNN model trained with RNA dataset versus baselines.*

| Method | Accuracy | AUC | F1 | $K_C$ |
|---|---|---|---|---|
| X2Graph (fusion) | **0.9242** | **0.9877** | **0.9098** | **0.8806** |
| X2Graph Reactome | 0.8990 | 0.9856 | 0.8796 | 0.8412 |
| X2Graph MoNDO | 0.90 t4 | 0.9854 | 0.8671 | 0.8430 |
| X2Graph BioGrid | 0.8995 | 0.9867 | 0.8804 | 0.8439 |
| MLP | 0.8805 | 0.9496 | 0.8502 | 0.8080 |
| TabPFN | 0.8769 | 0.9719 | 0.8434 | 0.8034 |
| XGBoost | 0.9194 | 0.9857 | 0.8975 | 0.8729 |
| LASSO | 0.9040 | 0.9821 | 0.8742 | 0.8490 |
| Ridge | 0.8826 | 0.8954 | 0.8567 | 0.8098 |

As shown in the above table, again the fusion model outperformed the baseline models for all metrics.

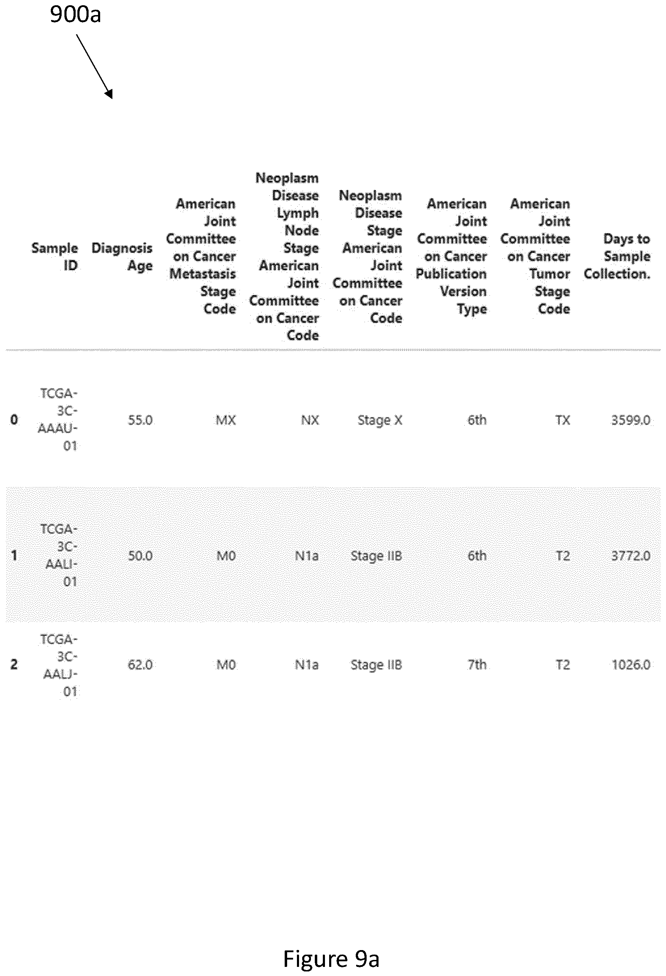

**Figure 9a** provides an example extract of a clinical dataset 900a which may be processed using the methods described herein. This example dataset contains a heterogeneous mix of 64 numerical, categorical, and ordinal features.

The categorical values may be converted into numerical values. The GNN model may be configured to accept numerical values rather than text. The dataset may also be normalized before graph creation. In this example the knowledge base was constructed for this dataset using verified and publicly available information about each feature, as well as the other features or factors which may influence feature values or status.

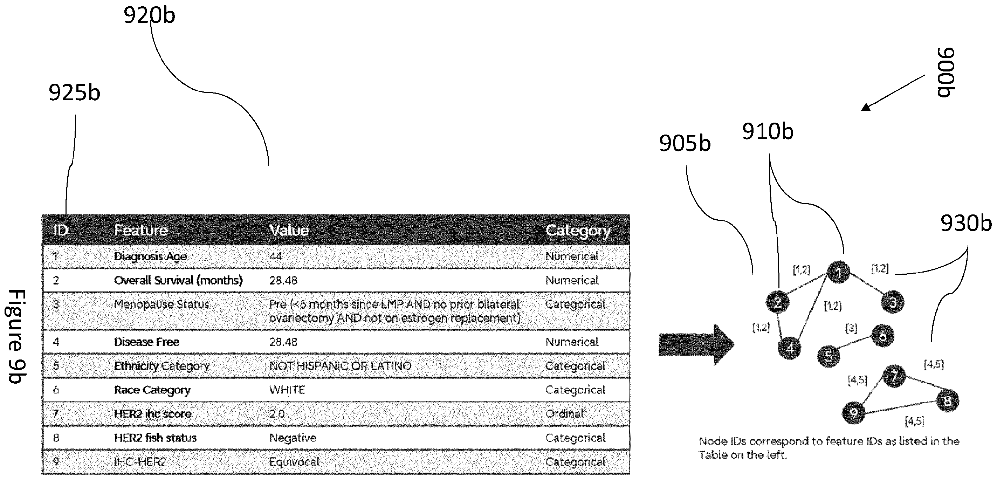

**Figure 9b** shows an illustrative example of the clinical tabular dataset to graph conversion process for a subset of the clinical dataset 900b. The index 910b inside each node of the graph 905b corresponds to the feature label (i.e., ID 925b) in the table 920b. The numbers on the edges 930b indicate the sources of knowledge and references used to establish those connections. In particular, the KB used to create the graph in Figure 9b were:

1. Chen, Hai-long, et al. "Effect of age on breast cancer patient prognoses: a population-based study using the SEER

18 database." PloS one 11.10 (2016): e0165409.
2. Park, Woong Ki, et al. "The Prognostic Impact of HER2-Low and Menopausal Status in Triple-Negative Breast Cancer." Cancers 16.14 (2024): 2566.
3. Nhim, Vutha, et al. "Racial/ethnic differences in the clinical presentation and survival of breast cancer by subtype." Frontiers in Oncology 14 (2024).
4. FISH and IHC Testing for HER2: Test Length, Results, and 7 More Facts To Know [FISH and IHC Testing for HER2: Test Length, Results, and 7 More Facts To Know | MyBCTeam, Accessed: 9/12/2024]
5. Ashfaq, Raheela, et al. "Correlation of HER2 expression by IHC, DNA Microarray and FISH in breast cancer." Cancer Research 71.8_Supplement (2011): 3143-3143.

The method described herein, in this instance using a single knowledge base, outperformed all baseline models in all categories apart from F1 score where it ranked second, as shown in Table 6 below.

*Table 6 Performance of GNN model trained with clinical dataset versus baselines.*

| Method | Accuracy | AUC | F1 | $K_C$ |
|---|---|---|---|---|
| X2Graph | **0.7487** | **0.8930** | 0.6523 | **0.5797** |
| TabPFN | 0.6735 | 0.8493 | 0.6073 | 0.4802 |
| MLP | 0.7043 | 0.8522 | 0.6168 | 0.5074 |
| XGBoost | 0.7250 | 0.8884 | **0.6800** | 0.5711 |
| LASSO | 0.6509 | 0.8349 | 0.6039 | 0.4624 |
| Ridge | 0.6522 | 0.7505 | 0.6083 | 0.4728 |

As shown in the examples above, the methods provided herein may convert measured tabular biological data into graphs, before using as input data for performing an application task. Such biological data tends to be more widespread in a tabular format. The methods may allow for converting all these data types into graphs, enabling their integration into the graph artificial intelligence technology. The methods described herein, for instance Figures 1, 2 and 6, and the architecture used with the methods, for instance Figure 5, may be applied to any suitable tabular data. Suitable tabular data may be data where relationships/connections between features may be established. The relationships between the features may guide the modelling process through inductive bias injection.

The methods described herein may be used for explainability and interpretability (XAI). As 1-D samples may be converted into graphs and modelled using GNN(s), graph-based XAI methods such as key nodes/edges/subgraph analysis may be applied to identify important features/connections/both. Furthermore, the methods may be employed for solutions in filling missing nodes or identifying hidden connections between features.

The results for CNV, RNA and Clinical datasets are shown in Tables 3, 5 and 6 respectively. While the tables show that the MLP outperforms LASSO and RIDGE on Clinical, it suffers a setback on datasets with small samples by features ratio such as CNV and RNA. TabPFN achieved better performance than MLP on several metrics - e.g. AUC on CNV and RNA, KC on CNV - but worse in others, especially on Clinical data. XGBoost, after being properly tuned, had the highest performance among baselines.

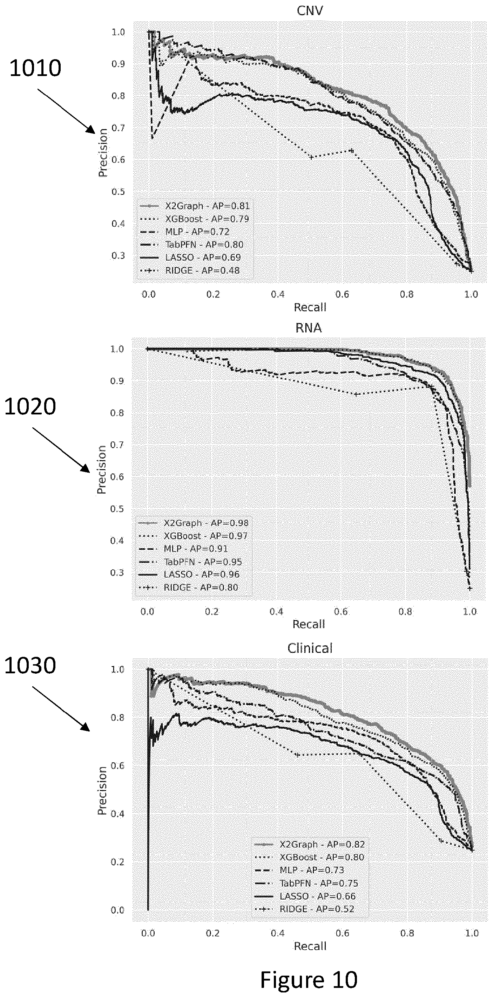

Finally, the methods proposed herein outperformed all other baseline models across various metrics, except for the F1 score on the Clinical datasets, where its performance was second to XGBoost on F1 but higher on accuracy, AUC and KC. For the CNV and RNA datasets, the methods for individual KBs consistently outperformed the MLP. This demonstrates that the strong inductive bias introduced by the KBs may be a key factor in reducing overfitting and, consequently, enhancing performance. The Precision-Recall (PR) curves and Average Precision (AP) scores in Figure 10 also depict the GNNs superiority versus other methods.

**Figure 10** shows Precision-Recall (PR) curves and Average Precision (AP) of the methods described herein using the GNN and baselines on the three benchmarks across the 10-fold cross-validation test sets. The Figure shows a CNV graph 1010 for the CNV dataset, an RNA graph 1020 for the RNA dataset and a clinical graph 1030 for the clinical dataset. The curves for RIDGE are different from the rest because RIDGE is a feature selection method which only outputs the predicted class instead of probabilities for each class.

**Figure 11** shows the k most importance features identified from the methods described herein for CNV 1110, RNA 1120 and Clinical 1130 data. A baseline comparison graph 1140 also shows fractions of these features evidenced in literature.

Interpretability: Feature importance. The inventors used GNNExplainer [Zhitao Ying, Dylan Bourgeois, Jiaxuan You, Marinka Zitnik, and Jure Leskovec. Gnnexplainer: Generating explanations for graph neural networks. NeurIPS, 32, 2019.*]* to identify the columns in each BioTD that contributed most to the GNN model predictions. For simplicity, a fold 0 model (out of 10-fold cross-validation) was used in testing. For each graph sample in the test set, GNNExplainer

approximates a subgraph that gives the closest prediction as the full graph. The selected nodes in the subgraphs are mapped back to the corresponding BioTD columns, thanks to its feature index. Figure 11 (a-b) shows the top 10 most important features for CNV and RNA, respectively (out of more than 20k original features available in the BioTD).

The selected features were further queried on UNIPROT [The UniProt Consortium. Uniprot: the universal protein knowledgebase in 2025. Nucleic Acids Research, 53(D1):D609-D617, 2025.] to find possible links with breast cancer (Query format: https://www.uniprot.org/citations?query=BREAST+ CANCER+AND+{*X*} where X is the gene of interest).

Several genes are also evidenced in literature for strong correlation with the disease; for example, TAZ is a breast cancer oncogene, G6PD is highly expressed in breast cancer tissues, and ADAM10 has recently been identified as a mediator for invasion and migration of breast cancer cells. The top 5 features for Clinical are also shown in Figure 11(c). The highest contributing features were ER/PR status by IHC which are clinically important markers for cancer treatment.

The fraction of top genes with links to breast cancer were also compared for the method disclosed herein for the GNN and other two popular baselines - XGBoost and MLP in Figure 11(d). For XGBoost, the genes used most often for splitting across all decision trees were identified. For MLP, Integrated Gradient [Mukund Sundararajan, Ankur Taly, and Qiqi Yan. Axiomatic attribution for deep networks. In ICML, pages 3319-3328. PMLR, 2017*]* was employed as the interpretability method. Figure 11(d) indicates that the GNN method may include more genes with evidenced links to breast cancer compared with the baselines (Clinical results are not shown as all features have a clear correlation with cancer. The graph-based interpretability method may therefore explain feature importance in a tabular dataset.

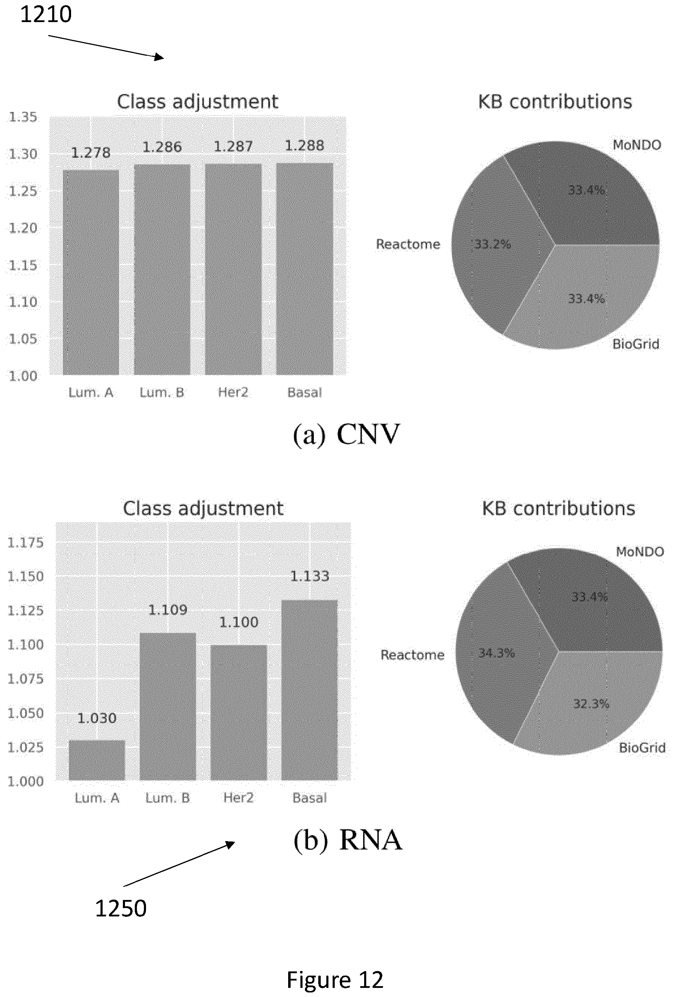

**Figure 12** shows contributions of each class in the classification of the medical condition and the contribution of the KB in multigraph fusion for (a) CNV 1210 and (b) RNA 1250, averaged across 10-fold cross validation.

Multigraph fusion: The contributions of each KB and class weight readjustment in the multigraph fusion models for CNV and RNA is shown in Figure 12. All 3 KBs are shown to contribute almost equally to the predictions of the fusion models. For CNV, the class weights were balanced, while in the case of RNA, the weight of the Luminal A class was adjusted slightly lower than that of others. This indicates a slight bias of RNA models for individual KBs towards Luminal A, which was corrected in the fusion model.

Ablation: The contributions of each X2Graph component to its overall performance was tested. Table 7 shows the AUC and F1 scores of the CNV GNN model using MoNDO as the KB. The inclusion of gene ID in the node vectors and pruning of the diploid nodes were ablated. Including gene ID for the GNN model may improve AUC by at least 15% and F1 score by at least 25% regardless of node pruning status.

The integration of gene ID may provide the context for the model to encode its corresponding gene values, especially when the position of nodes in a graph changes due to node pruning (by design) or node dropping (as part of training augmentation). On the other hand, node pruning may slightly improve the AUC/F1 performance, as shown by an increase of 1% if gene ID was included. This behaviour may be attributed to the stronger inductive bias brought by the pruning of diploid genes. The inventors found that when ID indexing is turned off, also turning off node pruning may improve performance so that the nodes appear in the same order across graphs, which may benefit learning in a no-context scenario.

The efficacy of the multi-graph fusion was evaluated with a modified version of the GNN model with a single graph model with each edge being a multidimensional vector corresponding to the number of KBs. This multi-edge GNN version may be considered as early fusion as opposed to the late fusion, as described in relation to multi-graph method described herein. It was found empirically that the multi-edge model underperformed the multi-graph, with AUC=0.8792 and F 1=0.6137 on CNV data (cf. Table 3). Given the limited data, late fusion, as in the multi-graph method described herein, may improve the inductive bias from multiple KBs for the GNN modelling.

*Table 7 Ablation of node pruning and gene ID indexing of the GNN model*

| AUC / F1 | | Node pruning ✓ | Node pruning ✗ |
|---|---|---|---|
| ID indexing | ✓ | **0.8892** / **0.6561** | 0.8771 / 0.6460 |
| | ✗ | 0.7068 / 0.3783 | 0.7363 / 0.3847 |

Thus, the methods described herein may provide efficient modelling methods and may be particularly effective for small tabular data. The GNN methods may introduce inductive bias to model training through predefined connections between table columns, effectively mitigating the overfitting problem when training with limited data. The results in the three above examples demonstrate the efficacy of the GNN model on three real-world biological datasets where prior knowledge about column connections is limited. Achieving state-of-the-art performance, the GNN models presents a deep learning alternative to traditional statistical and tree-based methods that have dominated tabular data analysis. This advancement may have the potential to significantly impact medicine and biology by enhancing the precision of cancer subtyping and other classification processes. The GNN model may be extended to other tabular data domains and

integrated it into multimodal learning frameworks, where samples from various modalities may be converted into graph representations.

**Figure 13** shows subgraph visualizations for three gene KBs, Mondo KB graph 1310, Reactome KB graph 1320 and BioGrid KB graph 1330. Each subgraph shows 1-hop neighbour connections centred at gene BRCA1.

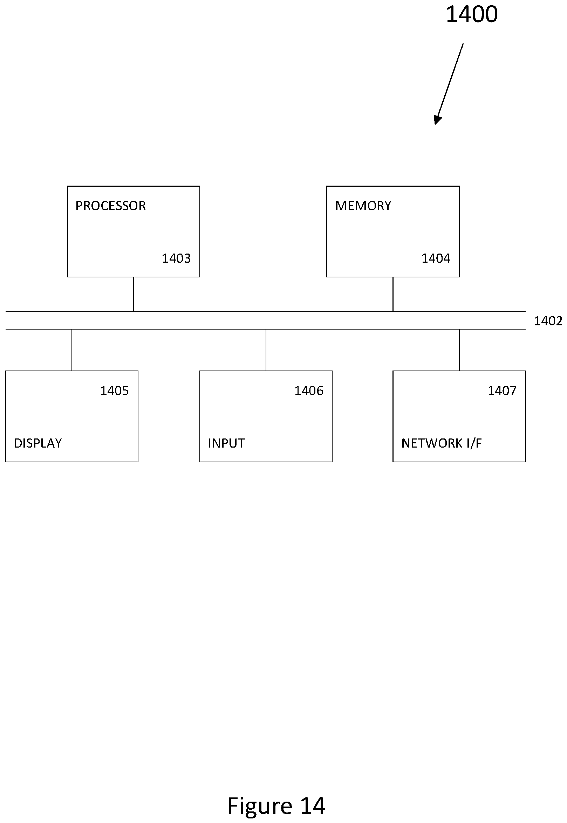

**Figure 14** is a block diagram of an information processing apparatus 1400 or a computing device 1400 or server 1400, such as a (data storage) server, which may be used to implement some or all of the operations of a method(s) described herein, and perform some or all of the tasks of apparatus of an embodiment. The computing device 1400 may be used to implement any of the method steps described above, e.g. any of steps S10-S40 and/or, for example.

The computing device 1400 comprises a processor 1403 and memory 1404. Optionally, the computing device also includes a network interface 1407 for communication with other such computing devices, for example with other computing devices of invention embodiments. Optionally, the computing device also includes one or more input mechanisms such as keyboard and mouse 1406, and a display unit such as one or more monitors 1405. These elements may facilitate user interaction. The components are connectable to one another via a bus 1402.

The memory 1404 may include a computer readable medium, which term may refer to a single medium or multiple media (e.g., a centralized or distributed database and/or associated caches and servers) configured to carry computer-executable instructions. Computer-executable instructions may include, for example, instructions and data accessible by and causing a computer (e.g., one or more processors) to perform one or more functions or operations. For example, the computer-executable instructions may include those instructions for implementing a method disclosed herein, or any method steps disclosed herein, for example any of steps S10-S40. Thus, the term "computer-readable storage medium" may also include any medium that is capable of storing, encoding or carrying a set of instructions for execution by the machine and that cause the machine to perform any one or more of the method steps of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 1403 is configured to control the computing device and execute processing operations, for example executing computer program code stored in the memory 1404 to implement any of the method steps described herein. The memory 1404 stores data being read and written by the processor 1403 and may store at least one biological dataset, knowledge base, and GNN, weights of a GNN, and/or any architecture and/or block described above, and/or programs for executing any of the method steps described above. These entities may be in the form of code blocks which are called when required and executed in a processor.

As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and operations discussed herein. The processor 1403 may be considered to comprise any of the blocks, or units, or modules, described herein. Any operations described as being implemented by a block may be implemented as a method by a computer and e.g. by the processor 1403.

The display unit 1405 may display a representation of data stored and/or generated by the computing device, such as classification of a medical condition. For instance, the computing device may be a device which accepts a sample as an input and determines a classification of a condition for the sample. The device may be request access to a website/web page or server by communicating with another computing device (e.g. a server). The other computing device may store instructions for performing the methods herein and the computing device may instruct the other computing device to provide a classification of the condition. The output may be shown as GUI windows and/or interactive representations enabling a user to interact with the apparatus 1400 by e.g. selection interaction, input text boxes, and/or any other output described above, and may also display a cursor and dialog boxes and screens enabling interaction between a user and the programs and data stored on the computing device. The input mechanisms 1406 may enable a user to input data and instructions to the computing device. For example, the display unit may display a GUI including a User panel, or input space, for the user to interact. For instance, a user may input a sample and query the classification of the sample. The user may interact with the GUI and display to generate and view a determined answer. Of course, the method may be performed automatically without interaction with a user.

The network interface (network I/F) 1407 may be connected to a network, such as the Internet, and is connectable to other such computing devices and/or servers via the network. The network I/F 1407 may control data input/output

from/to other apparatus via the network. Other peripheral devices such as microphone, speakers, printer, power supply unit, fan, case, scanner, trackerball etc may be included in the computing device.

Methods embodying the present invention may be carried out on a computing device/apparatus/server 1400 such as that illustrated in Figure 14. Such a computing device need not have every component illustrated in Figure 14 and may be composed of a subset of those components. For example, the apparatus 1400 may comprise the processor 1403 and the memory 1404 connected to the processor 1403. Or the apparatus 1400 may comprise the processor 1403, the memory 1404 connected to the processor 1403, and the display 1405. For example, the functions and/or architecture described in relation to Figures 2, 5 and 6 and the datasets in Figures 7-9b, may be stored in the memory and/or the method described in relation to Figure 1 may be stored in the memory. The processor may be configured to perform the method stored in the memory using, for example, the functions and/or architecture stored in the memory.

A method embodying the present invention may be carried out by a single computing device/server in communication with one or more (data storage) servers via a network. The computing device may be a data storage itself storing at least a portion of the data. The functions and/or architecture and/or method may be stored on the one or more data storage servers and the processor in the computing device may be configured to carry out the method steps.

A method embodying the present invention may be carried out by a plurality of computing devices operating in cooperation with one another. One or more of the plurality of computing devices may be a data storage server storing at least a portion of the data.

The invention may be implemented in digital electronic circuitry, or in computer hardware, firmware, software, or in combinations of them. The invention may be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a data processing environment. A computer program may be deployed to be executed on one module or on multiple modules at one site or distributed across multiple sites and interconnected by a communication network.

Method steps of the invention may be performed by one or more programmable processors executing a computer program to perform functions of the invention by operating on input data and generating output. Apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random-access memory or both. The essential elements of a computer are a processor for executing instructions coupled to one or more memory devices for storing instructions and data.

The above-described embodiments of the present invention may advantageously be used independently of any other of the embodiments or in any feasible combination with one or more others of the embodiments.

## Claims

**1.** A computer implemented method for training a graph neural network, GNN, to classify a medical condition of samples using a biological dataset, comprising:

loading the biological dataset, the biological dataset comprising samples and the samples comprising features and a classification of the medical condition;
extracting relationships between the features from a knowledge base;
constructing, for the samples, graphs comprising nodes and edges between the nodes, wherein the nodes correspond to the features and the edges correspond to the relationships; and
training the GNN with the graphs to provide the classification of the medical condition for the samples.

**2.** The method according to claim 1, wherein the features include feature labels, the dataset further includes attributes of the features and wherein the nodes comprise the feature labels and the attributes of the features.

**3.** The method according to claim 2, wherein the feature labels are gene labels, and the attributes are copy number variants, CNV, and/or RNA sequence values of the gene labels, and/or wherein the feature labels are clinical labels and the attributes are health record entries.

4. The method according to claims 2 or 3, wherein the feature labels are encoded using an embedding layer and the attributes are encoded using a linear layer.

5. The method according to any preceding claim, wherein the relationships between features indicate that the features are associated according to the knowledge base, preferably wherein the association between features comprises at least one of: the features sharing the same classification of the condition, the features sharing a same biological pathway group, and/or the features sharing a molecular interaction.

6. The method according to any preceding claim, wherein features present in both the biological dataset and the knowledge base are used to construct the graphs and preferably wherein any features not contained within the knowledge base are excluded from the graphs.

7. The method according to any preceding claim, wherein nodes and/or edges from one or more of the graphs are removed before the graphs are used to train the GNN.

8. The method according to any preceding claim, wherein the graphs form a graph set, and the method further comprises:

constructing another graph set using the features from the biological dataset and relationships from another knowledge base;
training another GNN with the other graph set; and
combining the classification of the condition of the GNN and the other GNN into a final classification of the condition, preferably wherein the classification of the condition is combined using a classifier, and more preferably wherein the classifier includes a linear layer.

9. The method according to any of claims 1-7, wherein further relationships between the features are extracted from another knowledge base and wherein the edges include the further relationships.

10. The method according to any preceding claim, wherein the knowledge base comprises further information on the relationships between features and wherein the edges include the further information as edge attributes.

11. The method according to any preceding claim, wherein the biological dataset is a tabular dataset.

12. The method according to any preceding claim, wherein the knowledge base is an external data source.

13. A computer implemented method for providing a classification of a medical condition from biological data, comprising:

inputting a new sample, the new sample comprising features;
loading a knowledge base, the knowledge base comprising relationships between the features in the sample;
constructing, for the sample, a graph comprising nodes and edges, wherein the features form the nodes and the relationships form the edges;
inputting the graph into a graph neural network, GNN, to classify the condition, wherein the GNN is trained according to any preceding claim; and
outputting the classification of the medical condition for the new sample.

14. A computer program which, when run on a computer, causes the computer to carry out a method for training a graph neural network, GNN, to classify a medical condition of samples using a biological dataset, comprising:

loading the biological dataset, the biological dataset comprising samples and the samples comprising features and a classification of the medical condition;
extracting relationships between the features from a knowledge base;
constructing, for the samples, graphs comprising nodes and edges between the nodes, wherein the nodes correspond to the features and the edges correspond to the relationships; and
training the GNN with the graphs to provide the classification of the medical condition for the samples.

15. An information processing apparatus for training a graph neural network, GNN, to classify a medical condition of samples using a biological dataset comprising a memory and a processor connected to the memory, wherein the processor is configured to:

load the biological dataset, the biological dataset comprising samples and the samples comprising features and a classification of the medical condition;
extract relationships between the features from a knowledge base;
construct, for the samples, graphs comprising nodes and edges between the nodes, wherein the nodes correspond to the features and the edges correspond to the relationships; and
train the GNN with the graphs to provide the classification of the medical condition for the samples.

**Amended claims in accordance with Rule 137(2) EPC.**

1. A computer implemented method for training a graph neural network (500), GNN, to classify a medical condition of samples using a biological dataset, comprising:

loading the biological dataset, the biological dataset comprising samples and the samples comprising features and each sample comprising a classification of the medical condition;
extracting relationships between the features from a knowledge base (350);
constructing, for the samples, graphs (300) comprising nodes (305) and edges (310) between the nodes, wherein the nodes correspond to the features and the edges correspond to the relationships, and wherein each graph corresponds to a sample; and
training the GNN with the graphs to provide the classification of the medical condition for the samples.

2. The method according to claim 1, wherein the features include feature labels, the dataset further includes attributes of the features and wherein the nodes (305) comprise the feature labels and the attributes of the features.

3. The method according to claim 2, wherein the feature labels are gene labels, and the attributes are copy number variants, CNV, and/or RNA sequence values of the gene labels, and/or wherein the feature labels are clinical labels and the attributes are health record entries.

4. The method according to claims 2 or 3, wherein the feature labels are encoded using an embedding layer and the attributes are encoded using a linear layer.

5. The method according to any preceding claim, wherein the relationships between features indicate that the features are associated according to the knowledge base (350), preferably wherein the association between features comprises at least one of: the features sharing the same classification of the condition, the features sharing a same biological pathway group, and/or the features sharing a molecular interaction.

6. The method according to any preceding claim, wherein features present in both the biological dataset and the knowledge base (350) are used to construct the graphs (300) and preferably wherein any features not contained within the knowledge base are excluded from the graphs.

7. The method according to any preceding claim, wherein nodes (305) and/or edges (310) from one or more of the graphs are removed before the graphs are used to train the GNN.

8. The method according to any preceding claim, wherein the graphs form a graph set, and the method further comprises:

constructing another graph set using the features from the biological dataset and relationships from another knowledge base;
training another GNN with the other graph set; and
combining the classification of the condition of the GNN and the other GNN into a final classification of the condition, preferably wherein the classification of the condition is combined using a classifier, and more preferably wherein the classifier includes a linear layer.

9. The method according to any of claims 1-7, wherein further relationships between the features are extracted from another knowledge base and wherein the edges include the further relationships.

10. The method according to any preceding claim, wherein the knowledge base comprises further information on the relationships between features and wherein the edges include the further information as edge attributes.

**11.** The method according to any preceding claim, wherein the biological dataset is a tabular dataset.

**12.** The method according to any preceding claim, wherein the knowledge base is an external data source.

**13.** A computer implemented method for providing a classification of a medical condition from biological data, comprising:

inputting a new sample, the new sample comprising features;
loading a knowledge base, the knowledge base comprising relationships between the features in the sample;
constructing, for the sample, a graph comprising nodes and edges, wherein the features form the nodes and the relationships form the edges;
inputting the graph into a graph neural network, GNN, to classify the condition, wherein the GNN is trained according to any preceding claim; and
outputting the classification of the medical condition for the new sample.

**14.** A computer program which, when run on a computer, causes the computer to carry out a method for training a graph neural network (500), GNN, to classify a medical condition of samples using a biological dataset, comprising:

loading the biological dataset, the biological dataset comprising samples and the samples comprising features and each sample comprising a classification of the medical condition;
extracting relationships between the features from a knowledge base (350);
constructing, for the samples, graphs (300) comprising nodes (305) and edges (310) between the nodes, wherein the nodes correspond to the features and the edges correspond to the relationships, and wherein each graph corresponds to a sample; and
training the GNN with the graphs to provide the classification of the medical condition for the samples.

**15.** An information processing apparatus for training a graph neural network (500), GNN, to classify a medical condition of samples using a biological dataset comprising a memory and a processor connected to the memory, wherein the processor is configured to:

load the biological dataset, the biological dataset comprising samples and the samples comprising features and each sample comprising a classification of the medical condition;
extract relationships between the features from a knowledge base (350);
construct, for the samples, graphs (300) comprising nodes (305) and edges (310) between the nodes, wherein the nodes correspond to the features and the edges correspond to the relationships, and wherein each graph corresponds to a sample; and
train the GNN with the graphs to provide the classification of the medical condition for the samples.

100

loading a biological dataset, the biological dataset comprising samples and the samples comprising features and a classification of the medical condition; — S10

extracting relationships between the features from a knowledge base — S20

constructing, for the samples, graphs comprising nodes and edges between the nodes, wherein the nodes correspond to the features and the edges correspond to the relationships — S30

training the GNN with the graphs to provide the classification of the medical condition for the samples. — S40

Figure 1

200
Dataset
210
210
Knowledge base
Graphs
220
GNN training
230
Prediction
240

Figure 2

300
302
f0
f1
f2
f3
f4
f5
f6
f7
f8...
x i-1
row x i
x i+1
0
-2
-1
+1
-1
+1
+2
-2
...
(.,-2)
(.,1)
(.,-1)
(.,1)
(.,2)
301
300
305
310
f0
f1
f3
f4
f5
f6
f8...
1
1
1
1
1
1
1
1
f0
f1
f3
f4
f5
f6
f8
Knowledge base (KB)
350

Figure 3

400
420
Dim=n
concat
Dim=n/2
Dim=n/2
Linear layer
Embedding layer
427
CNV value
Gene ID
425
410
Dim=2

Figure 4

500
510
520
522
Activation
525
530
532
Activation
535
540
Node embedding
550
Readout
560
Classification
570
A
B
C
D

Figure 5

600
Dataset
KB1
KB2
KB k
Graph
Graph
.......
Graph
GNN training
GNN training
.......
GNN training
Fusion model
610
Prediction
620

Figure 6

| | sample_id | ACAP3 | ACTRT2 | AGRN | ANKRD65 | ATAD3A | ATAD3B | ATAD3C | AURKAIP1 | B3GALT6 | ... | H2AFB3 | MPP1 | MTCP1 | RAB39B | SMIM9 | SNORA36A | SNORA56 | TMLHE | VBP1 | label |
|---|---|---|---|---|---|---|---|---|---|---|---|---|---|---|---|---|---|---|---|---|---|
| 0 | tcga-a8-a06y-01 | -1 | -1 | -1 | -1 | -1 | -1 | -1 | -1 | -1 | ... | -1 | -1 | -1 | -1 | -1 | -1 | -1 | -1 | -1 | 0 |
| 1 | tcga-e2-a15d-01 | 0 | 0 | 0 | 0 | 0 | 0 | 0 | 0 | 0 | ... | 1 | 1 | 1 | 1 | 1 | 1 | 1 | 1 | 1 | 0 |
| 2 | tcga-bh-a0de-01 | -1 | -1 | -1 | -1 | -1 | -1 | -1 | -1 | -1 | ... | 0 | 0 | 0 | 0 | 0 | 0 | 0 | 0 | 0 | 0 |
| 3 | tcga-e2-a1ig-01 | 0 | 0 | 0 | 0 | 0 | 0 | 0 | 0 | 0 | ... | 0 | 0 | 0 | 0 | 0 | 0 | 0 | 0 | 0 | 0 |
| 4 | tcga-a7-a3iy-01 | 0 | 0 | 0 | 0 | 0 | 0 | 0 | 0 | 0 | ... | 0 | 0 | 0 | 0 | 0 | 0 | 0 | 0 | 0 | 0 |
| 5 | tcga-a2-a0sv-01 | -1 | -1 | -1 | -1 | -1 | -1 | -1 | -1 | -1 | ... | 1 | 1 | 1 | 1 | 1 | 1 | 1 | 1 | 1 | 1 |
| 6 | tcga-a2-a04n-01 | 0 | 0 | 0 | 0 | 0 | 0 | 0 | 0 | 0 | ... | 0 | 0 | 0 | 0 | 0 | 0 | 0 | 0 | 0 | 0 |
| 7 | tcga-b6-a0wt-01 | 0 | 0 | 0 | 0 | 0 | 0 | 0 | 0 | 0 | ... | 0 | 0 | 0 | 0 | 0 | 0 | 0 | 0 | 0 | 0 |
| 8 | tcga-ol-a66h-01 | 0 | 0 | 0 | 0 | 0 | 0 | 0 | 0 | 0 | ... | 0 | 0 | 0 | 0 | 0 | 0 | 0 | 0 | 0 | 0 |
| 9 | tcga-c8-a135-01 | -1 | -1 | -1 | -1 | -1 | -1 | -1 | -1 | -1 | ... | 1 | 1 | 1 | 1 | 1 | 1 | 1 | 1 | 1 | 2 |


700
710
feature name
patient ID
feature value
target value
705
715
720


Figure 7

800

| | sample_id | LOC100130426 | UBE2Q2P3 | UBE2Q2P3.1 | HMGB1P1 | TIMM23 | MOXD2 | LOC155060 | RNU12-2P | SSX9 | ... | ZXDB | ZXDC | ZYG11A | ZYG11B | ZYX | FLJ10821 | ZZZ3 | TPTEP1 | AKR1C6P | label |
|---|---|---|---|---|---|---|---|---|---|---|---|---|---|---|---|---|---|---|---|---|---|
| 0 | tcga-a1-a0sd-01 | 0.0 | 3.394761 | 3.623410 | 5.936944 | 9.935043 | 0.0 | 6.404266 | 0.412294 | 0.0 | ... | 9.178121 | 10.260573 | 5.090959 | 9.763351 | 11.797934 | 10.321892 | 10.224725 | 6.443182 | 0.000000 | 0 |
| 1 | tcga-a1-a0se-01 | 0.0 | 3.464276 | 2.444296 | 7.268146 | 10.083915 | 0.0 | 6.242256 | 0.000000 | 0.0 | ... | 9.507031 | 9.994127 | 5.872570 | 9.909282 | 11.554980 | 9.856998 | 10.072055 | 9.760130 | 0.000000 | 0 |
| 2 | tcga-a1-a0sf-01 | 0.0 | 3.492071 | 3.553373 | 7.151710 | 10.253452 | 0.0 | 8.301259 | 0.000000 | 0.0 | ... | 9.061675 | 10.042267 | 6.580103 | 9.785005 | 12.383833 | 9.869360 | 8.991768 | 5.735121 | 0.892818 | 0 |
| 3 | tcga-a1-a0sg-01 | 0.0 | 2.820179 | 2.426962 | 6.120690 | 10.068524 | 0.0 | 7.997307 | 0.443607 | 0.0 | ... | 8.737701 | 10.111880 | 1.286881 | 9.458872 | 11.512063 | 10.727833 | 9.156148 | 4.731200 | 0.000000 | 0 |
| 4 | tcga-a1-a0sh-01 | 0.0 | 1.427713 | 2.001730 | 6.336289 | 9.951572 | 0.0 | 6.595525 | 0.000000 | 0.0 | ... | 9.185110 | 9.648174 | 4.382902 | 10.403267 | 12.416260 | 10.359519 | 9.968833 | 8.835339 | 0.000000 | 0 |

Figure 8

900a

| | Sample ID | Diagnosis Age | American Joint Committee on Cancer Metastasis Stage Code | Neoplasm Disease Lymph Node Stage American Joint Committee on Cancer Code | Neoplasm Disease Stage American Joint Committee on Cancer Code | American Joint Committee on Cancer Publication Version Type | American Joint Committee on Cancer Tumor Stage Code | Days to Sample Collection. |
|---|---|---|---|---|---|---|---|---|
| **0** | TCGA-3C-AAAU-01 | 55.0 | MX | NX | Stage X | 6th | TX | 3599.0 |
| **1** | TCGA-3C-AALI-01 | 50.0 | M0 | N1a | Stage IIB | 6th | T2 | 3772.0 |
| **2** | TCGA-3C-AALJ-01 | 62.0 | M0 | N1a | Stage IIB | 7th | T2 | 1026.0 |

Figure 9a

900b
905b
910b
920b
925b
930b
1
2
3
4
5
6
7
8
9
[1,2]
[1,2]
[1,2]
[1,2]
[3]
[4,5]
[4,5]
[4,5]
Node IDs correspond to feature IDs as listed in the Table on the left.

| ID | Feature | Value | Category |
|---|---|---|---|
| 1 | Diagnosis Age | 44 | Numerical |
| 2 | Overall Survival (months) | 28.48 | Numerical |
| 3 | Menopause Status | Pre (<6 months since LMP AND no prior bilateral ovariectomy AND not on estrogen replacement) | Categorical |
| 4 | Disease Free | 28.48 | Numerical |
| 5 | Ethnicity Category | NOT HISPANIC OR LATINO | Categorical |
| 6 | Race Category | WHITE | Categorical |
| 7 | HER2 ihc score | 2.0 | Ordinal |
| 8 | HER2 fish status | Negative | Categorical |
| 9 | IHC-HER2 | Equivocal | Categorical |

Figure 9b

1010
CNV
Precision
Recall
X2Graph - AP=0.81
XGBoost - AP=0.79
MLP - AP=0.72
TabPFN - AP=0.80
LASSO - AP=0.69
RIDGE - AP=0.48
1020
RNA
X2Graph - AP=0.98
XGBoost - AP=0.97
MLP - AP=0.91
TabPFN - AP=0.95
LASSO - AP=0.96
RIDGE - AP=0.80
1030
Clinical
X2Graph - AP=0.82
XGBoost - AP=0.80
MLP - AP=0.73
TabPFN - AP=0.75
LASSO - AP=0.66
RIDGE - AP=0.52


Figure 10

1110
1120
CNV top features
RAB39B
DKC1
CLIC2
IKBKG
G6PD
LAGE3
GDI1
FAM50A
ATP6AP1
TAZ
Has link with breast cancer
1.0
1.5
2.0
2.5
3.0
Importance score
RNA top features
ACTB
ABCD3
ABL1
ALG2
COL1A2
ADCY6
ACADVL
ADAM9
ACADSB
ADAM10
Has link with breast cancer
17
18
19
20
21
22
Importance score
(a) CNV
(b) RNA
Clinical top features
ER Status By IHC
PR status by ihc
Fraction Genome Altered
Neoplasm Disease Stage AJCC
Mutation Count
Has link with breast cancer
0
1
2
3
4
5
6
Importance score
Top Genes Relevance to Breast Cancer
Gene Relevance Score
0.7
0.6
0.5
0.4
0.3
0.2
0.1
0.0
CNV
RNA
X2Graph
XGBoost
MLP
(c) Clinical
(d) Baselines comparison
1130
1140

Figure 11

1210

Class adjustment
1.35
1.30
1.25
1.20
1.15
1.10
1.05
1.00
1.278
1.286
1.287
1.288
Lum. A
Lum. B
Her2
Basal
KB contributions
MoNDO
33.4%
Reactome
33.2%
33.4%
BioGrid

(a) CNV

Class adjustment
1.175
1.150
1.125
1.100
1.075
1.050
1.025
1.000
1.030
1.109
1.100
1.133
Lum. A
Lum. B
Her2
Basal
KB contributions
MoNDO
33.4%
Reactome
34.3%
32.3%
BioGrid

(b) RNA

1250

Figure 12

1310
MoNDO
1320
Reactome
1330
BioGrid
BRCA1
OPCML
PRKN
CHEK2
CDH1
TP53
BRIP1
KRAS
CASP8
HMMR
ATM
ESR1
PHB
PALB2
BARD1
BRCA2
AKT1

Figure 13

1400
PROCESSOR
1403
MEMORY
1404
1402
1405
DISPLAY
1406
INPUT
1407
NETWORK I/F

Figure 14

# EUROPEAN SEARCH REPORT

Application Number
EP 25 16 4204

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/249973 A2 (ILLUMINA INC [US]) 5 December 2024 (2024-12-05)<br>* paragraph [0009] *<br>* paragraph [0026] - paragraph [0030] *<br>* paragraph [0034] *<br>* paragraph [0037] *<br>* paragraph [0042] - paragraph [0043] *<br>* paragraph [0050] - paragraph [0051] *<br>* paragraph [0058] - paragraph [0060] *<br>* paragraph [0063] - paragraph [0064] *<br>* paragraph [0066] *<br>* paragraph [0068] - paragraph [0069] *<br>* paragraph [0072] - paragraph [0074] *<br>* paragraph [0082] *<br>* Facsimile page 52 *<br>* Facsimile page 60 *<br>----- | 1-15 | INV.<br>G16B40/20<br>G16H50/20 |
| X | US 2021/081717 A1 (CREED PAIDI [GB] ET AL) 18 March 2021 (2021-03-18)<br>* paragraph [0001] *<br>* paragraph [0004] *<br>* paragraph [0008] - paragraph [0009] *<br>* paragraph [0012] - paragraph [0014] *<br>* paragraph [0024] - paragraph [0025] *<br>* paragraph [0045] - paragraph [0046] *<br>* paragraph [0050] - paragraph [0051] *<br>* paragraph [0068] - paragraph [0069] *<br>* paragraph [0076] - paragraph [0077] *<br>* paragraph [0082] - paragraph [0083] *<br>* paragraph [0092] - paragraph [0094] *<br>* paragraph [0096] - paragraph [0097] *<br>* paragraph [0103] - paragraph [0104] *<br>* paragraph [0168] - paragraph [0171] *<br>-----<br>-/-- | 1-15 | TECHNICAL FIELDS SEARCHED (IPC)<br>G16B<br>G16H |

The present search report has been drawn up for all claims

1

| Place of search | Date of completion of the search | Examiner |
|---|---|---|
| Munich | 19 August 2025 | Martínez Cebollada |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 1 of 2

Europäisches Patentamt
European Patent Office
Office européen des brevets

# EUROPEAN SEARCH REPORT

Application Number
EP 25 16 4204

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/174217 A1 (PAI SUMIT [IE] ET AL) 10 June 2021 (2021-06-10)<br>* paragraph [0004] *<br>* paragraph [0029] - paragraph [0031] *<br>* paragraph [0060] - paragraph [0062] *<br>* paragraph [0064] - paragraph [0065] *<br>* paragraph [0067] - paragraph [0072] *<br>* paragraph [0092] *<br>* paragraph [0104] - paragraph [0105] *<br>* paragraph [0107] - paragraph [0108] *<br>----- | 1-15 | |

TECHNICAL FIELDS SEARCHED (IPC)

The present search report has been drawn up for all claims

| Place of search | Date of completion of the search | Examiner |
|---|---|---|
| Munich | 19 August 2025 | Martínez Cebollada |

1

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 25 16 4204

This annex lists the patent family members relating to the patent documents cited in the above-mentioned European search report. The members are as contained in the European Patent Office EDP file on
The European Patent Office is in no way liable for these particulars which are merely given for the purpose of information.

19-08-2025

| Patent document cited in search report | Publication date | Patent family member(s) | Publication date |
|---|---|---|---|
| WO 2024249973 A2 | 05-12-2024 | NONE | |
| US 2021081717 A1 | 18-03-2021 | CN 112119412 A | 22-12-2020 |
| | | EP 3794511 A1 | 24-03-2021 |
| | | US 2021081717 A1 | 18-03-2021 |
| | | WO 2019220128 A1 | 21-11-2019 |
| US 2021174217 A1 | 10-06-2021 | CN 112925857 A | 08-06-2021 |
| | | EP 3832487 A1 | 09-06-2021 |
| | | US 2021174217 A1 | 10-06-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 24191385


### Non-patent literature cited in the description


- **TIANQI CHEN** ; **CARLOS GUESTRIN**. Xgboost: A scalable tree boosting system.. *Proc. ACM SIGKDD*, 2016, 785-794
- **SHWARTZ-ZIV, R.** ; **ARMON, A.** Tabular data: Deep learning is not all you need.. *Information Fusion*, 2022, vol. 81, 84-90
- **MUNGALL et al.** The monarch initiative: an integrative data and analytic platform connecting phenotypes to genotypes across species.. *Nucleic acids research*, 2017, vol. 45 (D1), D712-D722
- **FABREGAT et al.** The reactome pathway knowledgebase.. *Nucleic acids research*, 2018, vol. 46 (D1), D649-D655
- **E. CERAMI** ; **J. GAO** ; **U. DOGRUSOZ** ; **B. E. GROSS** ; **S. O. SUMER** ; **B. A. AKSOY** ; **A. JACOBSEN** ; **C. J. BYRNE** ; **M. L. HEUER** ; **E. LARSSON et al.** The cbio cancer genomics portal: an open platform for exploring multidimensional cancer genomics data. *Cancer discovery*, 2012, vol. 2, 401
- **S. PARKER** ; **M. MULLINS** ; **M. C. CHEANG** ; **S. LEUNG** ; **D. VODUC** ; **T. VICKERY** ; **S. DAVIES** ; **C. FAURON** ; **X. HE** ; **Z. HU et al.** Supervised risk predictor of breast cancer based on intrinsic subtypes. *Journal of clinical oncology*, 2009, vol. 27, 1160
- **D. NETANELY** ; **A. AVRAHAM** ; **A. BEN-BARUCH** ; **E. EVRON** ; **R. SHAMIR**. Expression and methylation patterns partition luminal-a breast tumors into distinct prognostic subgroups,. *Breast Cancer Research*, 2016, vol. 18, 1
- Scalable and loosely-coupled multimodal deep learning for breast cancer subtyping. *Under review*, 2025
- **OUGHTRED et al.** The biogrid interaction database: 2019 update. *Nucleic acids research*, 2019, vol. 47 (D1), D529-D541
- **DANIELA FURRER** ; **SIMON JACOB,** ; **CHANTAL CARON** ; **FRANCOIS SANSCHAGRIN** ; **LOUISE PROVENCHER** ; **CAROLINE DIORIO**. Concordance of her2 immunohistochemistry and fluorescence in situ hybridization using tissue microarray in breast cancer.. *Anticancer Research*, 2017, vol. 37 (6), 3323-3329
- Scalable and loosely-coupled multimodal deep learning for breast cancer subtyping,. *Under review*, 2025
- **XINGZE WANG** ; **GUOXIAN YU** ; **ZHONGMIN YAN** ; **LIN WAN** ; **WEI WANG** ; **LIZHEN CUI.** Lung cancer subtype diagnosis by fusing image-genomics data and hybrid deep networks. *IEEE/ACM Trans. Comp. biology and bioinformatics,*, 2021, vol. 20 (1), 512-523
- **T LIU** ; **J HUANG** ; **T LIAO** ; **R PU** ; **S LIU** ; **PENG.** A hybrid deep learning model for predicting molecular subtypes of human breastcancer using multimodal data.. *Irbm*, 2022, vol. 43 (1), 62-74
- **NOAH HOLLMANN** ; **SAMUEL MULLER** ; **KATHARINA EGGENSPERGER** ; **FRANK HUTTER.** Tabpfn: A transformer that solves small tabular classification problems in a second.. *arXiv preprint arXiv:2207.01848,*, 2022
- **ROBERT TIBSHIRANI**. Regression shrinkage and selection via the lasso. Royal Stat.. *Society*, 1996, vol. 58 (1), 267-288
- **DONALD W MARQUARDT** ; **RONALD D SNEE**. Ridge regression in practice.. *The American Statistician*, 1975, vol. 29 (1), 3-20
- **NÚÑEZ-CARPINTERO, IKER et al.** he multilayer community structure of medulloblastoma.. *Iscience*, 2021, vol. 24, 4
- **CHEN, HAI-LONG et al.** Effect of age on breast cancer patient prognoses: a population-based study using the SEER 18 database.. *PloS one*, 2016, vol. 11 (10), e0165409
- **PARK, WOONG KI et al.** The Prognostic Impact of HER2-Low and Menopausal Status in Triple-Negative Breast Cancer. *Cancers*, 2024, vol. 16 (14), 2566

- **NHIM, VUTHA et al.** Racial/ethnic differences in the clinical presentation and survival of breast cancer by subtype.. *Frontiers in Oncology*, 2024, 14
- *FISH and IHC Testing for HER2: Test Length, Results, and 7 More Facts To Know [FISH and IHC Testing for HER2: Test Length, Results, and 7 More Facts To Know* | *MyBCTeam*, 09 December 2024
- **ASHFAQ, RAHEELA et al.** Correlation of HER2 expression by IHC, DNA Microarray and FISH in breast cancer. *Cancer Research*, 2011, 3143-3143
- **ZHITAO YING** ; **DYLAN BOURGEOIS** ; **JIAXUAN YOU** ; **MARINKA ZITNIK** ; **JURE LESKOVEC**. Gnnexplainer: Generating explanations for graph neural networks.. *NeurIPS*, 2019, 32
- The UniProt Consortium. Uniprot: the universal protein knowledgebase in 2025. *Nucleic Acids Research*, 2025, vol. 53 (D1), D609-D617
- **MUKUND SUNDARARAJAN** ; **ANKUR TALY** ; **QIQI YAN**. Axiomatic attribution for deep networks. *ICML*, 2017, 3319-3328